(19) Europäisches Patentamt
European Patent Office
Office européen des brevets

(11) Veröffentlichungsnummer: **0 017 209**
**B1**

(12) **EUROPÄISCHE PATENTSCHRIFT**

(45) Veröffentlichungstag der Patentschrift: **22.12.82**

(21) Anmeldenummer: **80101709.6**

(22) Anmeldetag: **31.03.80**

(51) Int. Cl.³: **C 07 D 487/04,** C 07 D 295/08, C 07 D 211/42, C 07 D 295/14, C 07 D 453/02, C 07 D 295/10, C 07 D 265/30, C 07 D 211/70, C 07 D 211/22, A 01 N 43/00 // C07C43/225 ,(C07D487/04, 209/00, 209/00)

# (54) Substituierte Alkylammoniumsalze, ihre Herstellung und Verwendung zur Regulierung des Pflanzenwachstums.

(30) Priorität: **04.04.79 DE 2913523**

(43) Veröffentlichungstag der Anmeldung: **15.10.80 Patentblatt 80/21**

(45) Bekanntmachung des Hinweises auf die Patenterteilung: **22.12.82 Patentblatt 82/51**

(84) Benannte Vertragsstaaten: **AT BE CH DE FR GB IT LU NL SE**

(56) Entgegenhaltungen:

**CH-A-422 758**
**DE-A-2 017 497**
**DE-A-2 206 267**
**GB-A-952 736**
**GB-A-982 572**
**US-A-3 255 196**

**J. Appl. Chem. USSR, 43, 2073-74, 1970**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Sauter, Hubert, Dr., Neckarpromenade, D-6800 Mannheim 1 (DE)**
Erfinder: **Zeeh, Bernd, Dr., Thorwaldsenstrasse 5, D-6700 Ludwigshafen (DE)**
Erfinder: **Buschmann, Ernst, Dr., An der Froschlache 7, D-6700 Ludwigshafen (DE)**
Erfinder: **Jung, Johann, Dr. Dipl.-Landw., Hardenburgstrasse 19, D-6703 Limburgerhof (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

EP 0 017 209 B1

# Substituierte Alkylammoniumsalze, ihre Herstellung und Verwendung zur Regulierung des Pflanzenwachstums

Die Erfindung betrifft neue substituierte Alkylammoniumsalze, nämlich bestimmte Aryloxy- und Arylthioalkylammoniumsalze, Mittel zur Regulierung des Pflanzenwachstums, die diese Verbindungen enthalten, die Verwendung dieser Verbindungen und Mittel zur Regulierung des Pflanzenwachstums sowie Verfahren zur Herstellung der neuen Verbindungen.

Es ist aus der Literatur schon eine Anzahl heterocyclischer quartärer Aryloxyalkylammoniumsalze mit antibakterieller, nematozider oder pharmazeutischer Wirkung bekannt [zum Beispiel: GB-PS 952 736, GB-PS 863 197, GB-PS 982 572, DE-OS 2 234 080, Z. Prikl. Chim. 43, 2057 (1970) und J. Appl. Chem. USSR 43, 2073 (1970)]. Ferner sind einige derartige Verbindungen auch schon für technische Zwecke, z.B. als Egalisierhilfsmittel zum Färben von Polyacrylnitril vorgeschlagen worden (DE-OS 2 206 267). Bei diesen Angaben findet sich jedoch kein Hinweis auf die pflanzenwachstumsregulierende Wirkung solcher Verbindungen. Es ist ferner vorgeschlagen worden, gewisse quartäre Phenoxyethylammoniumsalze, deren quartärer Stickstoff jedoch nicht Teil eines Heterocyclus ist, als Mittel zur Regulierung des Pflanzenwachstums zu verwenden (DE-OS 2 017 497). Deren Wirkung ist jedoch vor allem bei niedrigen Aufwandmengen nicht immer ausreichend.

Andererseits sind quartäre Ammoniumverbindungen von ganz anderer Struktur als Pflanzenwachstumsregulatoren beschrieben, z. B. das 2-Chlorethyl-trimethylammoniumchlorid (CCC, US-PS 3 156 544). Bei diesen Verbindungen handelt es sich jedoch nicht um quartäre Aryloxyalkylammoniumsalze oder Arylthioalkylammoniumsalze; ihre Wirkung ist auch, insbesondere bei niedrigen Aufwandmengen, nicht immer befriedigend.

Gegenstand der Erfindung sind substituierte Alkylammoniumsalze der Formel I

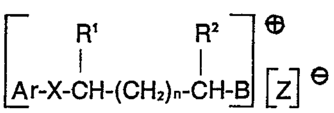

(I)

in der

Ar Phenyl bedeutet, das durch eine Trifluoromethyl-, Nitro-, Cyano-, $C_2$-$C_4$-Alkylcarbonyl-, $C_2$-$C_4$-Alkylcarbonylaminogruppe oder durch 2 bis 3 gleiche oder verschiedene der folgenden Substituenten substituiert ist: Alkyl, Alkenyl, Alkinyl, Cycloalkyl, Alkoxy, Alkenyloxy, Alkinyloxy, Alkylthio, Halogenalkyl, Halogenalkoxy, Halogenalkylthio, Alkylcarbonylamino, Alkylsulfonylamino, Alkylsulfonyl, Alkylaminosulfonyl, Dialkylaminosulfonyl mit jeweils bis zu 6 C-Atomen, Fluor, Chlor, Brom, Jod, Nitro, Cyano, Aminosulfonyl, Phenyl oder Benzyl,

X Sauerstoff oder Schwefel darstellt,

n die Zahl 0, 1 oder 2 ist,

$R^1$ und $R^2$ gleich oder verschieden sind und Wasserstoff oder $C_1$-$C_4$-Alkyl bedeutet,

B einen Chinuclidin- oder Pyrrolizidinbicyclus oder die Gruppe 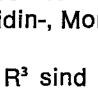 darstellt, in welcher

Y für eine gegebenenfalls durch 1 bis 3 gleiche oder verschiedene $C_1$-$C_4$-Alkyl-, -Alkoxy-, -Halogenalkyl-, Chlor-, Brom-, Hydroxy- oder Cyanoreste substituierte $-(CH_2)_4-$, $-(CH_2)_5-$, $-(CH_2)_6-$, $-(CH_2)_7-$, $-CH_2-CH=CH-(CH_2)_2-$, $-(CH_2)_2-O-(CH_2)_2-$, $-(CH_2)_2-S-(CH_2)_2-$, $-NH-(CH_2)_4-$ oder $-NH-CH_2-CH=CH-CH_2-$Gruppe steht und

$R^3$ geradkettiges oder verzweigtes $C_1$-$C_6$-Alkyl, -Alkenyl oder -Alkinyl bedeutet, das durch Halogen, Hydroxy, Cyano, $C_1$-$C_4$-Alkoxy oder -Alkylcarbonyl substituiert sein kann und

Z das Anion einer beliebigen, nicht phytotoxischen Säure HX bedeutet.

Ar bedeutet beispielsweise 3-Trifluoromethylphenyl, 4-Trifluoromethylphenyl, 2,4-Dichlorphenyl, 3,4-Dichlorphenyl, 3,5-Dichlorphenyl, 2,6-Dichlorphenyl, 2,4,5-Trichlorphenyl, 2,4,6-Trichlorphenyl, 2-Chlor-4-fluorphenyl, 2-Chlor-4-bromphenyl, 2-Methyl-4-chlorphenyl, 2-Chlor-4-phenylphenyl, 2-Benzyl-4-chlorphenyl, 3,5-Diethylphenyl, 2,4,6-Trimethylphenyl, 2,6-Dichlor-4-nitrophenyl, 2,6-Diiod-4-cyanophenyl, 2-Methoxy-4-methylphenyl, 4-Cyanophenyl, 3-Nitrophenyl, 2-Methyl-4-methylthiophenyl, 4-Chlor-2-methylphenyl, 4-Nitro-3-trifluoromethylphenyl, 2-Chlor-4-nitrophenyl, 3,5-Dimethoxyphenyl, 3-Acetylaminophenyl.

Bevorzugt sind Verbindungen der Formel I, in denen X=O, $R^1=R^2=H$ und n=0 bedeuten.

In einer weiteren Gruppe bevorzugter Verbindungen steht B für den Pyrrolizidinbicyclus oder für einen am quartären N durch $R^3$ substituierten Pyrrolidin-, Piperidin-, Morpholin- oder Hexamethylenimin-Ring.

Bevorzugte Reste $R^3$ sind Methyl, Ethyl, Propyl, Butyl, Allyl, Propargyl, 2-Chlorethyl, 2-Bromethyl, Cyanomethyl, Methylcarbonylmethyl, 2-Chlorpropen-3-yl, 2-Methylpropen-3-yl und 2-Buten-1-yl.

Da die Wirkung der erfindungsgemässen Verbindungen der Formel I auf das Kation zurückzuführen ist, kann das Anion $Z^{\ominus}$ aus den nicht phytotoxischen Säuren beliebig ausgewählt werden. $Z^{\ominus}$ bedeutet beispielsweise Acetat, Methylsulfonat, p-Toluolsulfonat, p-Dodecylbenzolsulfonat, Nitrat, Phosphat, Iodid, Sulfat, Methosulfat und insbesondere Chlorid oder Bromid. Das Anion $Z^{\ominus}$ wird normalerweise durch die Wahl des Quaternierungsmittels der Formel II bzw. V bestimmt; andere Anionen $Z^{\ominus}$ können jedoch anschliessend leicht durch Ionenaustausch nach allgemein bekannten Verfahren eingeführt werden (siehe z. B. Houben-Weyl, Methoden der Organischen Chemie, Band 11/2, S. 620-626 sowie Band 1/1, S. 544, Thieme Verlag, Stuttgart 1958).

Weiter betrifft die Erfindung ein Verfahren zur Herstellung substituierter Alkylammoniumsalze der Formel I, welches darin besteht, dass man in an sich bekannter Weise

a) Verbindungen der Formel II

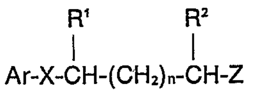

(II)

in der Ar, X, $R^1$, n und $R^2$ die oben angegebene Bedeutung haben und Z für eine nucleofuge Abgangsgruppe steht, mit einem tertiären Amin der Formel III

$$B \qquad \text{(III)}$$

in der B die oben angegebenen Bedeutungen hat, umsetzt oder

b) Verbindungen der Formel IV

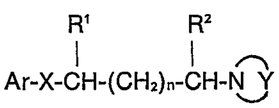

(IV)

in der Ar, X, $R^1$, n, $R^2$ und Y die oben genannten Bedeutungen haben, mit Alkylierungsmitteln der Formel V

$$R^3\text{-}Z \qquad \text{(V)}$$

in der $R^3$ die oben angegebene Bedeutung hat und Z für eine nucleofuge Abgangsgruppe steht, zum Quartärsalz der Formel I alkyliert.

Als nucleofuge Abgangsgruppe für das Verfahren a) eignen sich insbesondere Chlor, Brom, Jod, Methylsulfonat oder p-Toluolsulfonat. Die Umsetzung der Verbindungen II und III kann kann in Gegenwart oder Abwesenheit eines Lösungs- oder Verdünnungsmittels, wie Wasser oder die gebräuchlichen organischen Lösungsmittel, z. B. Kohlenwasserstoffe, Halogenkohlenwasserstoffe, Ketone, Alkohole, Ether, Nitrile, Ester und Dimethylformamid, in homogener oder inhomogener Phase bei 20-150°C, vorzugsweise 50-120°C, erfolgen. Die Reaktionspartner II und III können in beliebigen Mengenverhältnissen eingesetzt werden, bevorzugt sind equimolekulare Mengen oder ein Überschuss an Amin III.

Die Verbindungen der Formel II sind allgemein bekannte Verbindungen oder können leicht nach bekannten Verfahren hergestellt werden, z. B. durch Monoalkylierung von Phenolen ArOH oder Thiophenolen ArSH mit aliphatischen Dihalogeniden, wie 1,2-Dibromethan, 1,3-Dibrompropan oder 1,4-Dibrombutan, bevorzugt in Wasser oder besonders bevorzugt in siedendem Diethylketon oder Cyclopentanon in Gegenwart von mindestens equivalenten Mengen Kaliumcarbonat, (siehe Houben-Weyl, Methoden der Organischen Chemie, Band 6/3, S. 54-59, Thieme-Verlag, Stuttgart 1965 sowie die Beispiele 1 und 3).

Als tertiäre Amine der Formel III können beispielsweise N-Methylpyrrolidin, N-Ethylpyrrolidin, N-Allylpyrrolidin, N-Propylpyrrolidin, N-Butylpyrrolidin, N-Methylpiperidin, N-Methylhexamethylenimin, 2,4,6-Trimethylmorpholin, Chinuclidin oder Pyrrolizidin eingesetzt werden.

Für das Verfahren b) können als Alkylierungsmittel der Formel V beispielsweise Methylchlorid, Methylbromid, Methyliodid, Dimehtylsulfat, Allylbromid, Propargylchlorid, Isopentylbromid, Chloraceton, Chloracetonitril oder 2-Methoxyethyltosylat eingesetzt werden. Die Durchführung der Alkylierung erfolgt ohne Verdünnungs- bzw. Lösungsmittel oder in Gegenwart eines Verdünnungs- bzw. Lösungsmittels, wie Wasser, Ethanol, Aceton, Acetonitril, Ethylacetat, Ether, Toluol oder Dimethylformamid, in homogener oder inhomogener Phase dei Temperaturen zwischen 0 und 150°C, vorzugsweise 20 und 120°C. Die Mengenverhältnisse der Reaktionspartner können dabei in weiten Grenzen variiert werden, bevorzugt sind equimolekulare Verhältnisse oder ein bis zu 10facher molarer Überschuss des Alkylierungsmittels (siehe Houben-Weyl, Methoden der Organischen Chemie, Band 11/2, S. 591-601, Thieme Verlag, Stuttgart 1958, sowie die Beispiele 2 und 4). Als nucleofuge Abgangsgruppen eignen sich dieselben wie für das Verfahren a).

Die tertiären Amine der Formel IV sind bekannte Verbindungen oder nach bekannten Verfahren leicht zugänglich, z. B. nach dem Schema

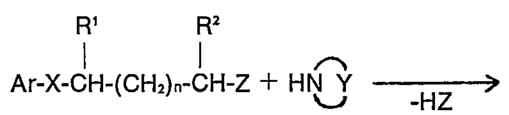


II VI IV

durch Alkylierung sekundärer Amine der Formel VI, in der Y die obengenannte Bedeutung hat, mit den oben erwähnten Verbindungen der Formel II. Die Reaktionsbedingungen entsprechen hierbei denen der Umsetzung nach dem Schema II + III → I, wobei hier ein 2- bis 10fach molarer Überschuss des Amins der Formel VI bevorzugt ist. Das bei der Reaktion entstandene HX kann leicht z. B. durch Behandeln des Produktgemisches mit wässrigen Alkalihydroxiden oder durch Abfiltrieren eines gegebenenfalls abgeschiedenen Säureadditionssalzes 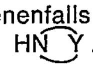 . HZ entfernt werden (siehe Beispiel 2).

Eine weitere an sich bekannte Methode zur Synthese tertiärer Amine der Formel IV besteht in der Umsetzung von Phenolen oder Thiophenolen der Formel VII oder deren Alkalisalzen, in denen Ar und X die oben angegebene Bedeutung haben, mit tertiären Aminen der Formel VIII oder deren Säureadditionssalzen, in denen $R^1$, n, $R^2$ und Y die obengenannten Bedeutungen haben und in denen Z für eine nucleofuge Abgangsgruppe, bevorzugt Chlor, Brom, Jod, Methansulfonat oder p-Toluolsulfonat steht (siehe Houben-Weyl, Methoden der Organischen Chemie, Band 6/3, S. 54-59, Thieme Verlag, Stuttgart 1965) sowie Beispiel 4 nach dem Schema:

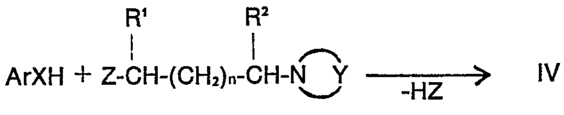


VII VIII

Als Amine der Formel VIII können beispielsweise N-(2-Chlorethyl)-pyrrolidin, N-(2-Chlorethyl)-piperidin, N-(2-Chlorpropyl)-pyrrolidin oder N-(2-Chlorethyl)-hexamethylenimin eingesetzt werden.

Für die Synthese von Zwischenprodukten der Formeln II und IV können beispielsweise die folgenden Phenole und Thiophenole eingesetzt werden:

2,4-Dichlorphenol, 2,4-Dichlorthiophenol, 3,4-Dichlorphenyl, 3,4-Dichlorthiophenol, 3,5-Dichlorphenol, 2,6-Dichlorphenol, 2,4,5-Trichlorphenol, 2,4,5-Trichlorthiophenol, 2,4,6-Trichlorphenol, 2,-3,4-Trichlorthiophenol, 2-Chlor-4-fluorphenol, 2-Fluor-4-chlorphenol, 2,6-Dichlor-4-fluorphenol, 2,4-Dibromphenol, 2,4,6-Tribromphenol, 2-Brom--4-chlorphenol, 4-Brom-2-chlorphenol, 4-Brom--2,6-dichlorphenol, 4-Brom-2,6-dichlorphenol, 4-Cyano-2,6-diiodphenol, 2-Trifluormethylphenol, 3-Trifluormethylphenol, 4-Trifluormethylphenol, 4-Nitro-3-trifluormethylphenol, 2,4-Dimethylphenol, 3,4-Dimethylphenol, 3,5-Dimethylphenol, 2,-4,6-Trimethylphenol, 3,5-Diethylphenol, 3,5-Diisopropylphenol, 2-Chlor-4-methylphenol, 4-Chlor-2--methylphenol, 2,4-Dimethylthiophenol, 3,4-Dichlor-2,6-dimethylphenol, 2,4-Dichlor-6-methylphenol, 4-Chlor-3-methylphenol, 2,6-Dimethyl-4--nitrophenol, 2,4-Dimethyl-6-nitrophenol, 2-Methyl-4-(methylthio)-phenol, 3-Methyl-4-(methylthio)-phenol, 4-Methyl-2-methoxyphenol, 2-Benzyl-4-chlorphenol, 2-Chlor-4-phenylphenol, 4-Brom-2-phenylphenol, 2-Nitrophenol, 3-Nitrophenol, 4-Nitrophenol, 2,4-Dinitrophenol, 2-Chlor-4--Nitrophenol, 4-Chlor-2-Nitrophenol, 2,6-Dichlor--4-nitrophenol, 4-Cyanophenol, 3-Cyanophenol, 2-Cyanophenol, 3-Acetylaminophenol, 4-Formylaminophenol, 4-Acetylaminophenol, 2-Acetylphenol, 3-Acetylphenol, 3-Butyrylphenol, 4-Acetylphenol.

Die folgenden Beispiele erläutern die Herstellung der erfindungsgemässen Verbindungen der Formel I und der zu ihrer Herstellung nötigen Zwischenprodukte der Formeln II und IV.

Beispiel 1

a) Herstellung des Ausgangsmaterials

Eine Mischung von 162 g 3-Trifluormethylphenol, 570 g 1,2-Dibromethan, 500 ml Cyclopentanon und 166 g trockenem, pulverisiertem Kaliumcarbonat wird unter Rühren 24 Stunden unter Rückfluss erhitzt. Sodann destilliert man Cyclopentanon und überschüssiges Dibromethan im Vakuum ab und nimmt den Rückstand in 500 ml Methylenchlorid und 200 ml 10%iger Natronlauge auf. Nach Abtrennen der wässrigen Phase wird die organische Schicht noch dreimal mit je 200 ml 10%iger Natronlauge und einmal mit 150 ml Wasser gewaschen, anschliessend über Natriumsulfat getrocknet und im Vakuum eingeengt. Der ölige Rückstand (174 g) enthält das 1-Brom-2-(3-trifluoromethylphenoxy)--ethan.

IR (Film): 1590, 1490, 1444, 1325, 1291, 1280, 1165, 1120, 1094, 1062, 790, 780, 693 $cm^{-1}$.

b) Herstellung des Endprodukts

10 g 1-Brom-2-(3-trifluoromethylphenoxy)-ethan und 20 g Pyrrolizidin werden 6 Stunden lang auf 100C. erhitzt. Nach dem Abkühlen wird die Mischung mit 10 ml Aceton und 15 ml Ether versetzt. Das abgeschiedene Öl kristallisiert bei −15°C durch. Man isoliert 8 g weisse Kristalle N-2-(3-Trifluoromethylphenoxy)-ethyl-pyrrolizidiniumbromid, die mit Ether gewaschen werden. Fp. 55-57°C.

Beispiel 2

a) Herstellung des Ausgangsmaterials

54 g 1-Brom-2-(3-trifluoromethylphenoxy)--ethan, 100 ml Toluol und 45 g Heptamethylenimin werden 24 Stunden unter Rückfluss erhitzt. Nach dem Abkühlen wird der Niederschlag abfiltriert und das Filtrat zweimal mit je 100 ml 10%iger wässriger Natriumhydroxidlösung und anschliessend fünfmal mit je 100 ml Wasser extrahiert, sodann über Natriumsulfat getrocknet und im Vakuum eingeengt. Der ölige Rückstand (46 g) enthält das N-2-(3-Trifluoromethylphenoxy)-ethylheptamethylenimin.

IR (Film): 1590, 1490, 1444, 1325, 1291, 1280, 1165, 1120, 1094, 1062, 790, 780, 693 $cm^{-1}$.

b) Herstellung des Endprodukts

Die Mischung aus 9,0 g N-2-(3-Trifluoromethylphenoxy)-ethyl-heptamethylenimin und 18,0 g Allylbromid wird 11 Stunden unter Rückfluss gekocht. Nach Abziehen des überschüssigen Allylbromids im Vakuum nimmt man den Rückstand in 10 ml Aceton auf. Aus dieser Lösung scheiden sich bei −15°C 7,0 g bräunliche Kristalle von N-Allyl-N-2-(3-trifluoromethylphenoxy)--ethyl-heptamethyleniminiumbromid ab, die mit Ethylacetat gewaschen werden. Fp. 119-125°C.

Beispiel 3

a) Herstellung des Ausgangsmaterials

Die Mischung aus 8,0 g Natriumhydroxid in 80 ml Wasser, 32 g 2,4,6-Trimethylphenol und 215 g 1,4-Dibrombutan wird 100 Stunden unter Rühren zum Rückfluss erhitzt. Nach dem Abkühlen wird die wässrige Phase abgetrennt und mit 200 ml Methylenchlorid extrahiert. Die vereinigten organischen Phasen werden dann zehnmal mit je 150 ml Wasser ausgeschüttelt, über Natriumsulfat getrocknet und durch Einengen im Vakuum von Methylenchlorid befreit. Den Rückstand destilliert man im Vakuum; nach einem Vorlauf, der aus überschüssigem 1,4-Dibrombutan besteht, gehen bei 110-130°C/2-4 mmHg 24 g 1-Brom-4-(2,4,6-trimethylphenoxy)--butan über.

IR (Film): 2910, 2860, 1483, 1442, 1374, 1308, 1245, 1216, 1147, 1039, 854 $cm^{-1}$.

b) Herstellung des Endprodukts

Die Mischung aus 7,0 g 1-Brom-4-(2,4,6-trimethylphenoxy)-butan und 20 g N-Methylpiperidin wird 8 Stunden lang zum Rückfluss erhitzt. Die auf 60°C abgekühlte Mischung verrührt man mit 20 ml Aceton, wobei sich beim weiteren Abkühlen ein Kristallbrei abscheidet, der nach Zusatz von 20 ml Ether abfiltriert und mit Toluol und Aceton gewaschen wird. Man erhält 8,0 g blassgelbe Kristalle N-Methyl-N-4-(2,4,6-trimethylphenoxy)-btuylpiperidiniumbromid vom Fp. 171-173°C.

Beispiel 4

a) Herstellung des Ausgangsmaterials

Zur Mischung von 100 g Natriumhydroxid und 160 g 2,4,5-Trichlorthiophenol in 2400 ml Wasser wird unter Stickstoff und unter Rühren eine Lösung von 85 g N-(2-Chlorethyl)-pyrrolidiniumhydrochlorid zugetropft und anschliessend 8 Stunden unter Rückfluss weitergekocht. Nach dem Abkühlen wird der Niederschlag abgesaugt, in 1000 ml Methylenchlorid aufgenommen und zweimal mit je 200 ml 10%iger wässriger Natriumhydroxidlösung sowie einmal mit 200 ml Wasser gewaschen. Nach dem Trocknen der organischen Phase über Natriumsulfat und Abdestillieren des Methylenchlorids im Vakuum erhält man 147 g N-2-(2,4,5-Trichlorphenylthio)-ethyl-pyrrolidin vom Schmelzpunkt 72-74°C.

b) Herstellung des Endprodukts

Die Mischung aus 15,5 g N-2-(2,4,5-Trichlorphenylthio)-ethyl-pyrrolidin, 12,5 g 2-Bromethanol und 20 ml Acetonitril wird 14 Stunden unter Rückfluss erhitzt und nach dem Abkühlen mit 100 ml Ether versetzt. Der entstandene Niederschlag wird abgesaugt und mit Ether gewaschen. Man erhält 18,3 g Kristalle N-2-Hydroxyethyl-N-2-(2,4,5-trichlorphenylthio)-ethyl-pyrrolidiniumbromid vom Schmelzpunkt 135-137°C.

Als weitere Beispiele für die erfindungsgemässen Verbindungen der Formel I seien die folgenden einzeln aufgeführt, deren Strukturen grösstenteils durch $^1$H-Kernresonanz und IR-Spektren bestätigt wurden:

5. 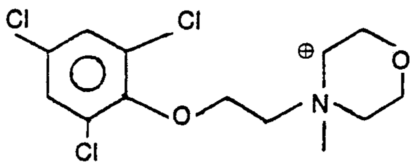 Br⊖ Fp. 104-108°C

6. 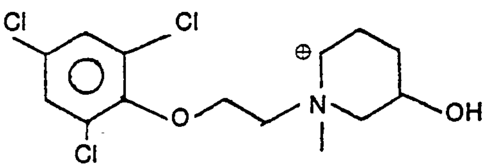 Br⊖ Fp. 140-143°C

7. 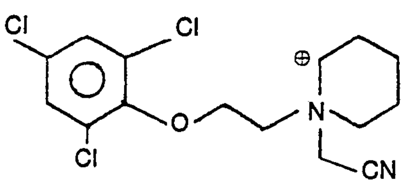 Cl⊖ Fp. 121-125°C

8. 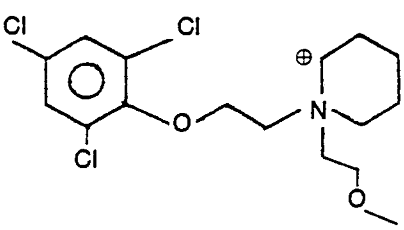 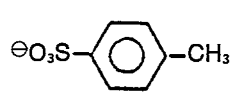 Fp. 88-89°C

9. 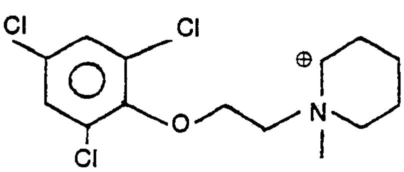 ⊖$O_3SOCH_3$ Fp. 135-142°C

| | | | |
|---|---|---|---|
| 10. | 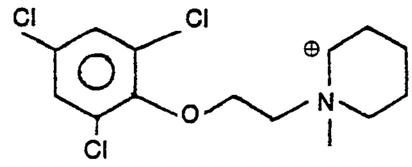 | Br⊖ | Fp. 198-200°C |
| 11. | 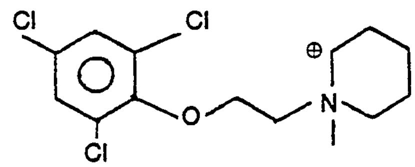 | J⊖ | Fp. 136-139°C |
| 12. | 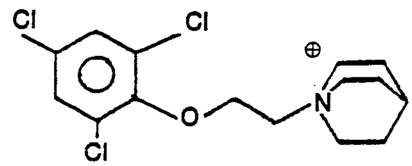 | Br⊖ | Fp. 178-180°C |
| 13. | 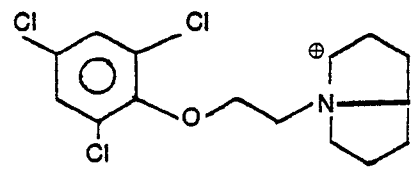 | Br⊖ | Fp. 141-144°C |
| 14. | 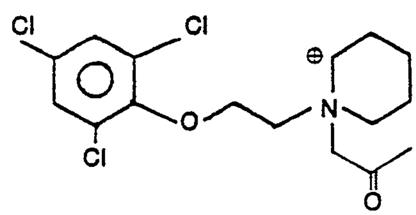 | Cl⊖ | Fp. 75-77°C |
| 15. | 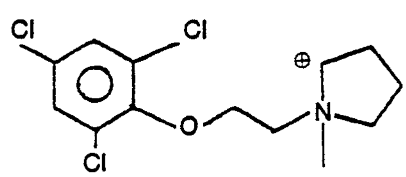 | Br⊖ | Fp. 62-65°C |
| 16. | 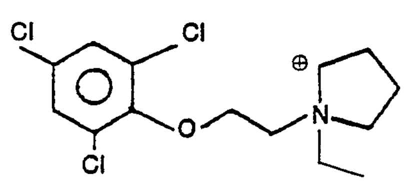 | Br⊖ | Fp. 116-118°C |

17. 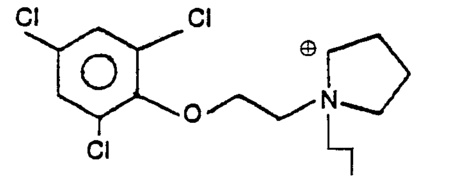 Br$^{\ominus}$ Fp. 177-178°C

18. 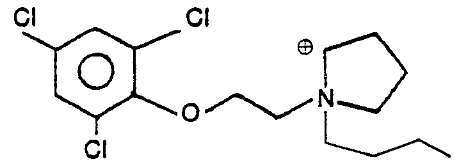 Br$^{\ominus}$ Fp. 165-168°C

19. 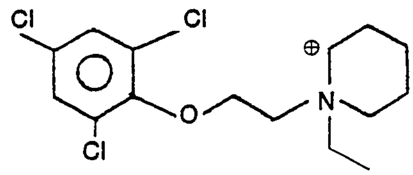 Br$^{\ominus}$ Fp. 150-152°C

20. 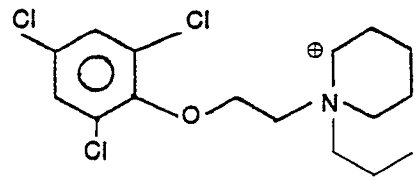 Br$^{\ominus}$ Fp. 183-184°C

21. 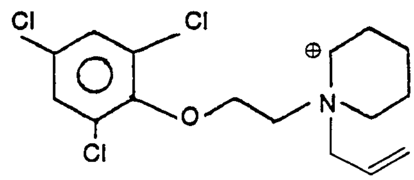 Br$^{\ominus}$ Fp. 155-156°C

22. 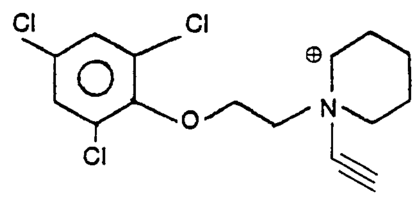 Br$^{\ominus}$ Fp. 184-187°C

23. 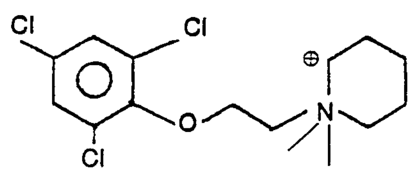 Br$^{\ominus}$ Fp. >200°C

| | | | |
|---|---|---|---|
| 24. | 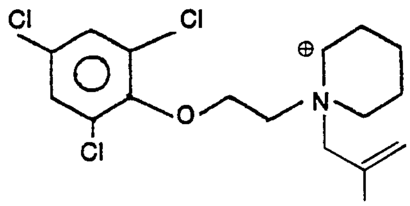 | $Cl^{\ominus}$ | Fp. 176-177°C |
| 25. | 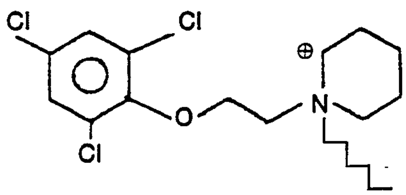 | $Br^{\ominus}$ | |
| 26. | 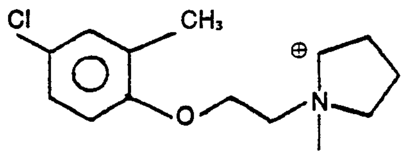 | $Br^{\ominus}$ | Fp. 58-61°C |
| 27. | 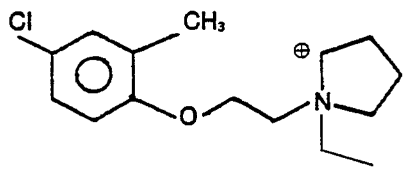 | $Br^{\ominus}$ | Fp. 165-167°C |
| 28. | 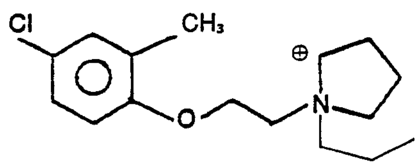 | $Br^{\ominus}$ | Fp. 104-107°C |
| 29. | 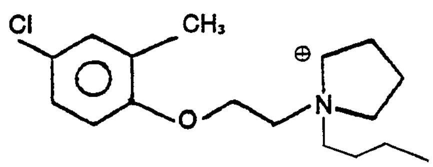 | $Br^{\ominus}$ | Fp. 101-103°C |
| 30. | 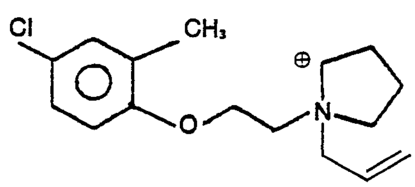 | $Br^{\ominus}$ | Fp. 138-139°C |

31. 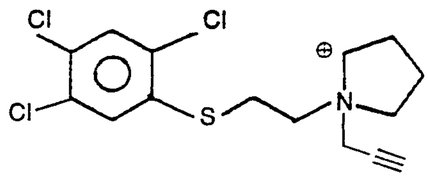 $Br^{\ominus}$ Fp. 106-109°C

32. 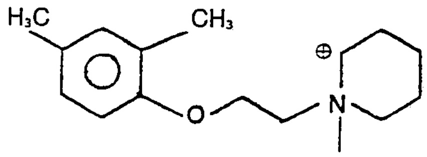 $Br^{\ominus}$ Fp. 145-147°C

33. 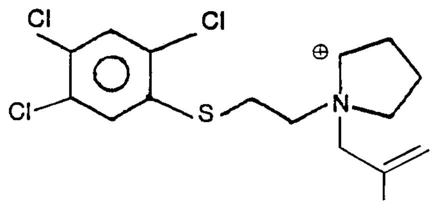 $Cl^{\ominus}$ Fp. 83-87°C

34. 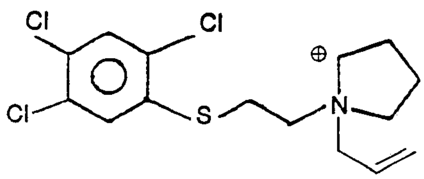 $Cl^{\ominus}$ Fp. 55-58°C

35. 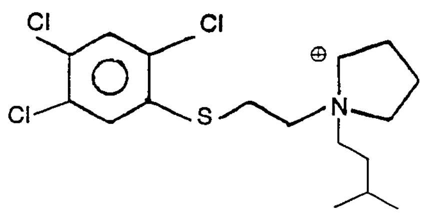 $Br^{\ominus}$ Fp. 130-133°C

36. 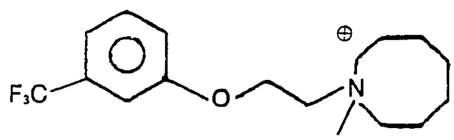 $Br^{\ominus}$ Fp. 179-181°C

37. 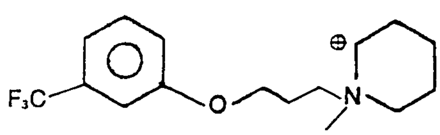 $Br^{\ominus}$ Fp. 156-157°C

38.

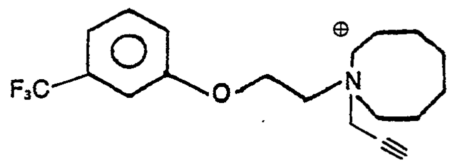

$Br^{\ominus}$

39.

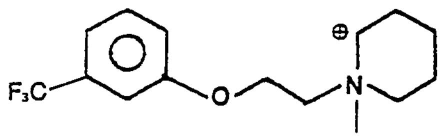

$Br^{\ominus}$ Fp. 83-87°C

40.

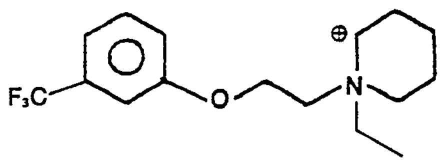

$Br^{\ominus}$ Fp. 110-112°C

41.

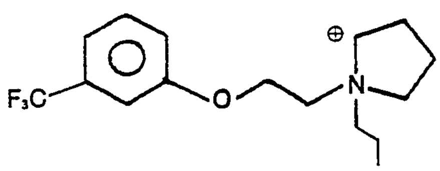

$Br^{\ominus}$ Fp. 137-139°C

42.

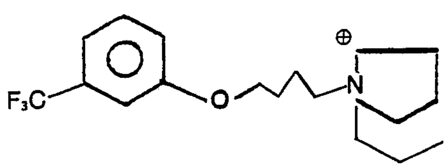

$Br^{\ominus}$ Fp. 83-85°C

43.

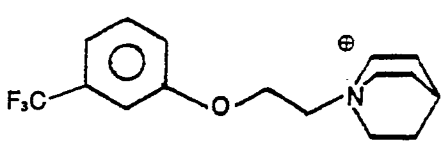

$Br^{\ominus}$ Fp. 168-170°C

44.

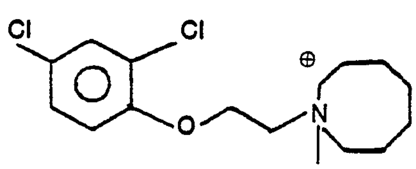

$Br^{\ominus}$ Fp. 140-143°C

45. 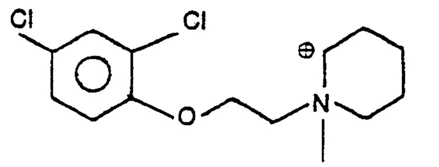 Br$^{\ominus}$ Fp. 181-182°C

46. 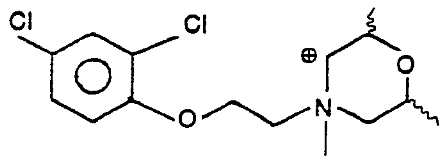 Br$^{\ominus}$ Fp. 204-209°C (cis-trans-Gemisch)

47. 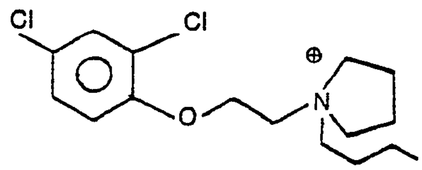 Br$^{\ominus}$ Fp. 131-134°C

48. 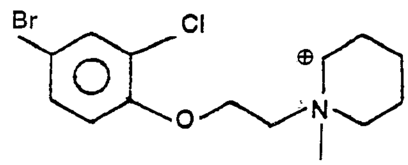 Br$^{\ominus}$ Fp. 200-202°C

49. 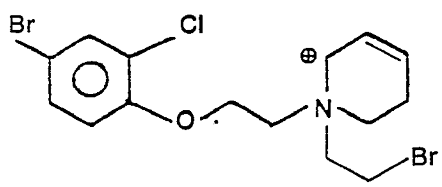 Br$^{\ominus}$ Fp. 155-159°C

50. 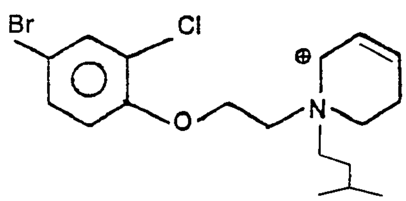 Br$^{\ominus}$ Fp. 139-143°C

51. 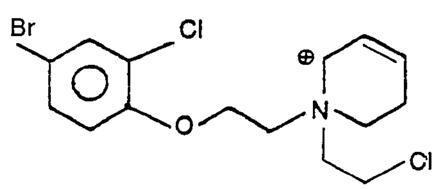 Br$^{\ominus}$ Fp. 141-144°C

52. 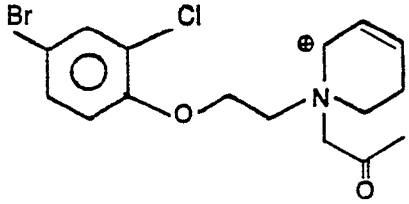 $Cl^{\ominus}$ Fp. 188-189°C

53. 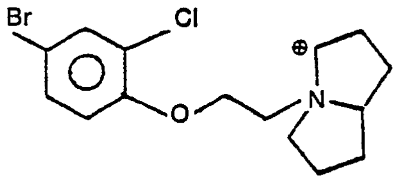 $Br^{\ominus}$ Fp. 91-93°C

54. 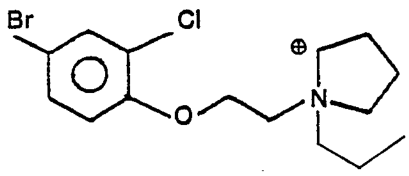 $Br^{\ominus}$ Fp. 153-154°C

55. 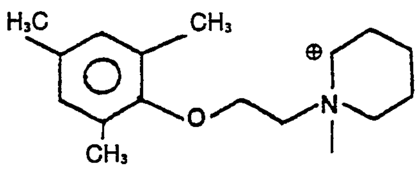 $Br^{\ominus}$ Fp. 193-195°C

56. 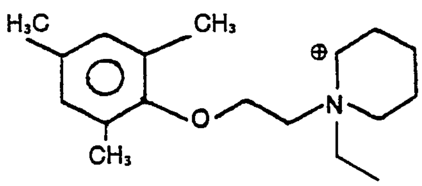 $Br^{\ominus}$ Fp. 176-178°C

57. 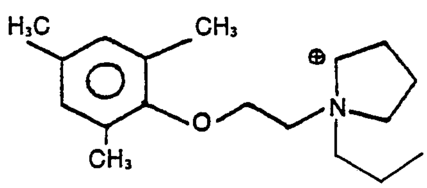 $Br^{\ominus}$ Fp. 187-188°C

58. 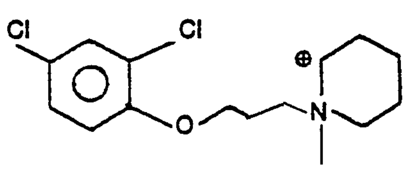 $Br^{\ominus}$ Fp. 148-151°C

| | | | |
|---|---|---|---|
| 59. | 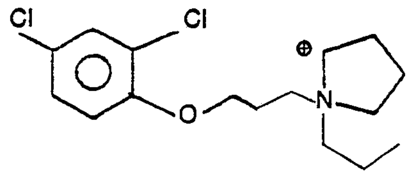 | Br$^{\ominus}$ | Fp. 122-127°C |
| 60. | 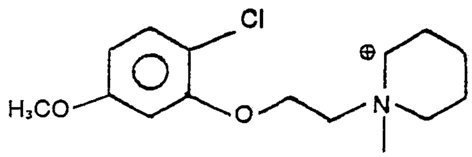 | Br$^{\ominus}$ | Fp. 144-148°C |
| 61. | 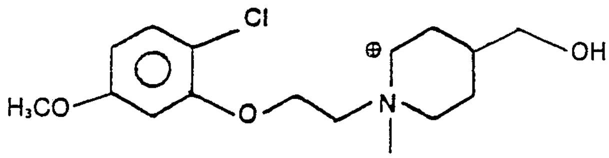 | Br$^{\ominus}$ | Fp. 160-161°C |
| 62. | 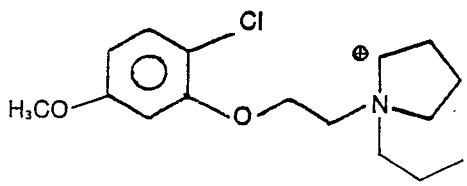 | Br$^{\ominus}$ | Fp. 146-148°C |
| 63. | 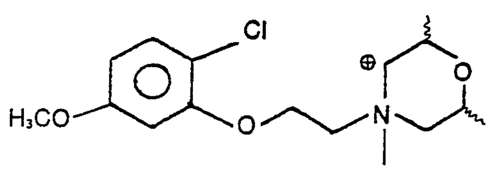 | Br$^{\ominus}$ | Fp. 162-165°C (cis-trans-Gemisch) |
| 64. | 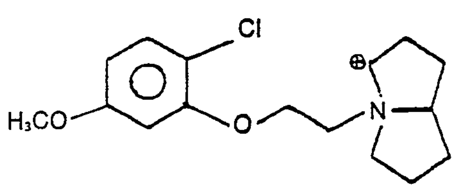 | Br$^{\ominus}$ | Fp. 125-126°C |
| 65. | 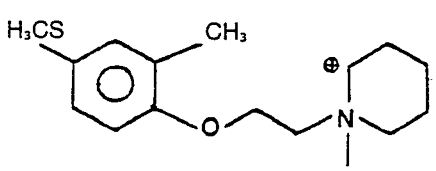 | Br$^{\ominus}$ | Fp. 69-73°C |

| | | | |
|---|---|---|---|
| 66. | 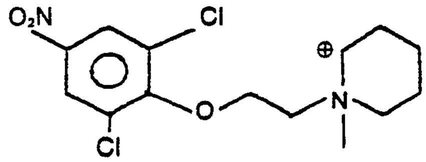 | $Br^{\ominus}$ | Fp. 163-165°C |
| 67. | 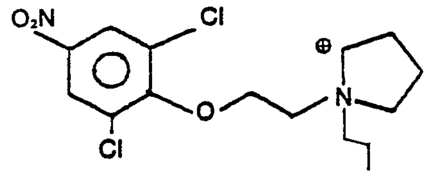 | $Br^{\ominus}$ | Fp. 62-66°C |
| 68. | 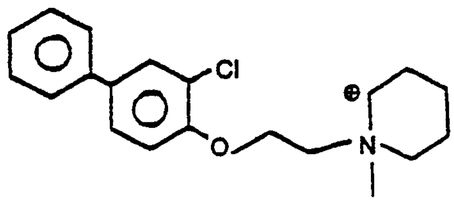 | $Br^{\ominus}$ | Fp. 158-161°C |
| 69. | 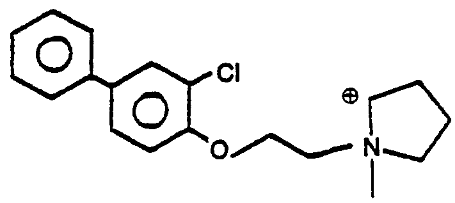 | $Br^{\ominus}$ | Fp. 152-156°C |
| 70. | 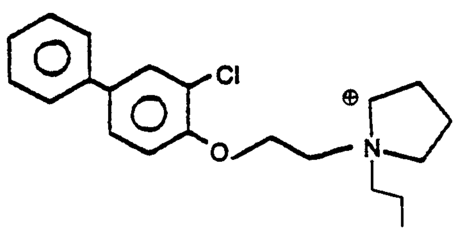 | $Br^{\ominus}$ | |
| 71. | 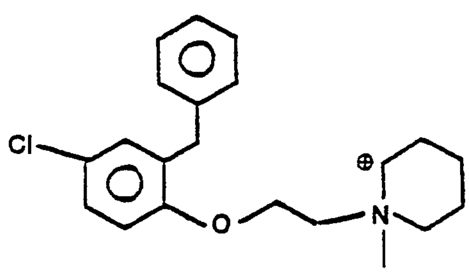 | $Br^{\ominus}$ | Fp. 145-147°C |
| 72. | 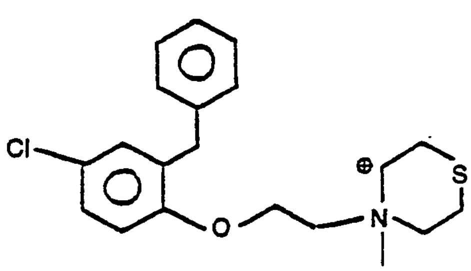 | $Br^{\ominus}$ | Fp. 183-186°C |

73.

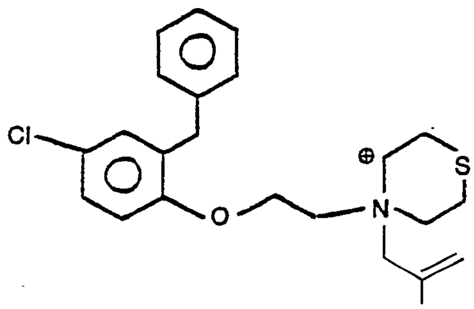

Cl⊖

74.

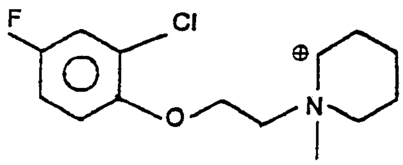

Br⊖ Fp. 157-162°C

75.

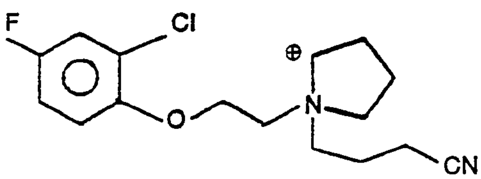

Br⊖ IR (Film): 2940, 2238, 1586, 1484, 1252, 1183, 1050, 900, 878, 851, 800, 752 $cm^{-1}$

76.

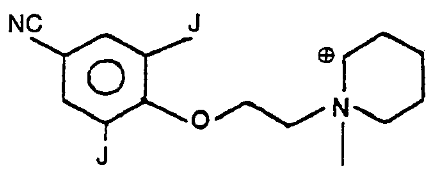

Br⊖ Fp. 176-180°C

77.

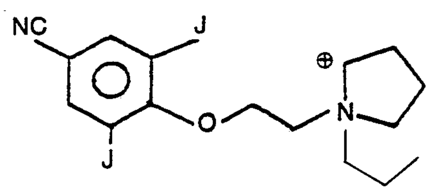

Br⊖ Fp. 164-170°C

78.

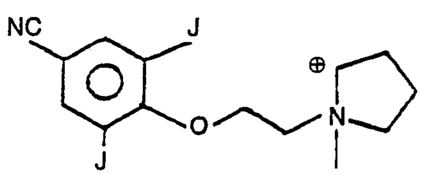

Br⊖

79.

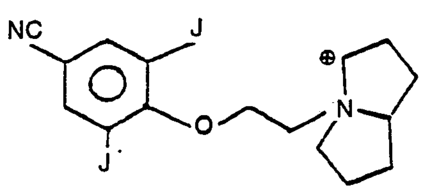

Br⊖

80.

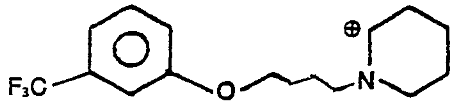

Br$^{\ominus}$ Fp. 123-125°C

81.

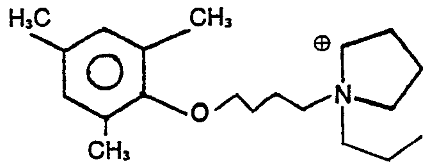

Br$^{\ominus}$ Fp. 160-163°C

82.

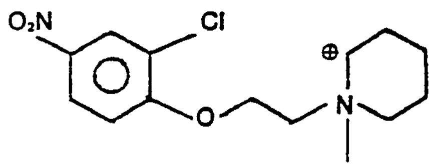

Br$^{\ominus}$ Fp. 109-113°C

83.

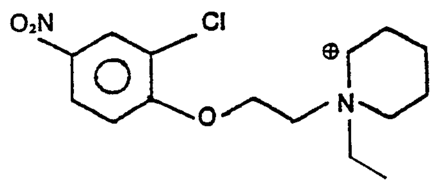

Br$^{\ominus}$ Fp. 170-174°C

84.

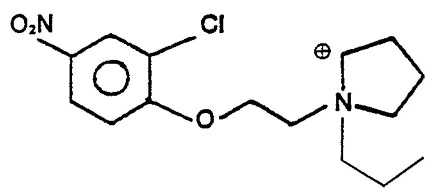

Br$^{\ominus}$ Fp. 165-170°C

85.

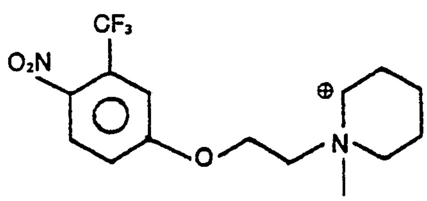

Br$^{\ominus}$ Fp. 179-180°C

86.

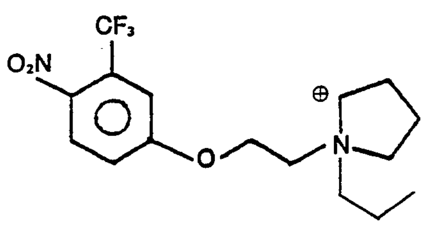

Br$^{\ominus}$ Fp. 121-123°C

| | | | |
|---|---|---|---|
| 87. | 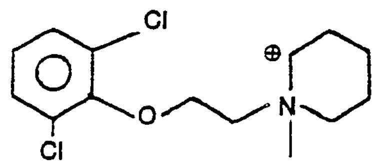 | Br$^{\ominus}$ | Fp. $>$200°C |
| 88. | 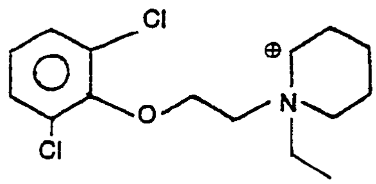 | Br$^{\ominus}$ | Fp. 79-80°C |
| 89. | 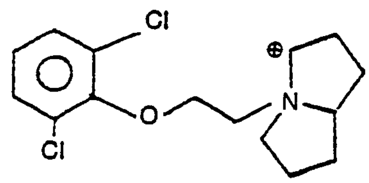 | Br$^{\ominus}$ | Fp. 50-56°C |
| 90. | 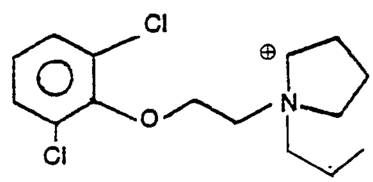 | Br$^{\ominus}$ | Fp. 219-220°C |
| 91. | 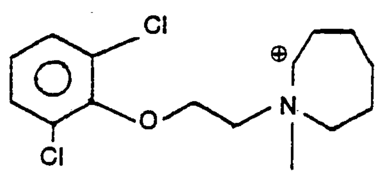 | Br$^{\ominus}$ | Fp. $>$200°C |
| 92. | 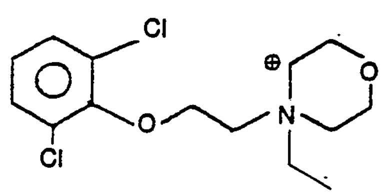 | Br$^{\ominus}$ | Harz |
| 93. | 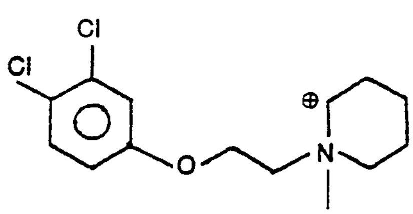 | Br$^{\ominus}$ | Fp. 146-149°C |

94. 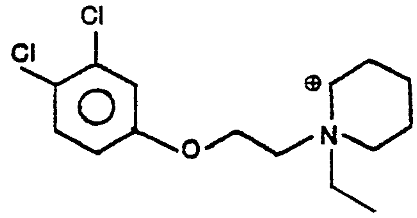 Br$^{\ominus}$ Fp. 136-139°C

95. 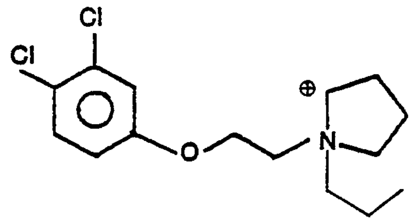 Br$^{\ominus}$ Fp. 139-142°C

96. 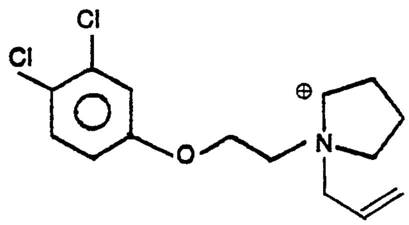 Br$^{\ominus}$ Fp. 173-177°C

97. 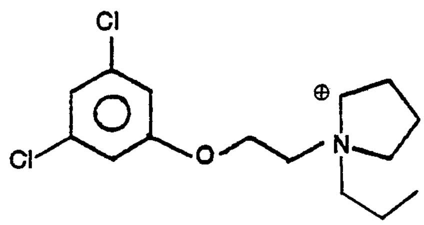 Br$^{\ominus}$ Fp. 115-117°C

98. 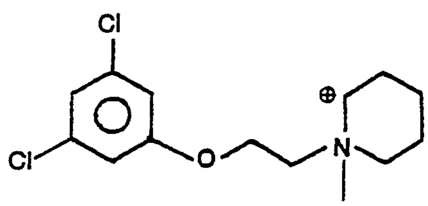 Br$^{\ominus}$ Fp. 166-168°C

99. 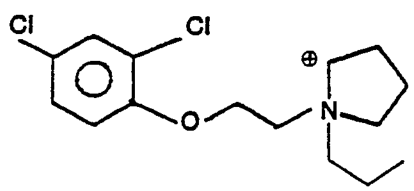 Br$^{\ominus}$ Fp. 144-145°C

100. 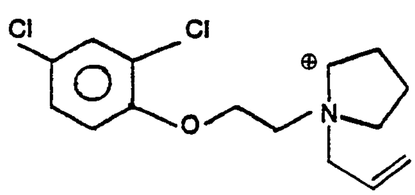 Br$^{\ominus}$ Fp. 142-143°C

| | | | |
|---|---|---|---|
| 101. | 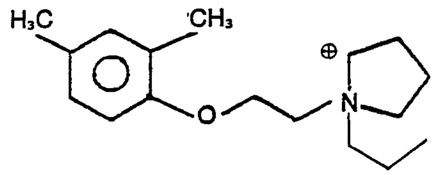 | Br$^{\ominus}$ | Fp. 120-125°C |
| 102. | 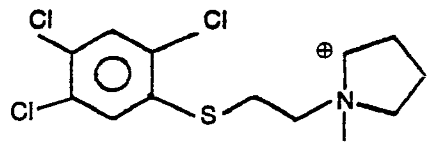 | Br$^{\ominus}$ | Fp. 80-81°C |
| 103. | 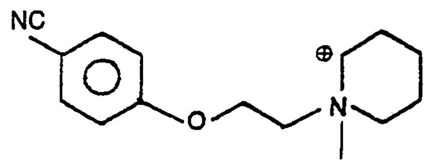 | Br$^{\ominus}$ | Fp. 82-85°C |
| 104. | 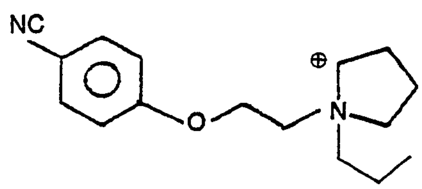 | Br$^{\ominus}$ | Fp. 98-103°C |
| 105. | 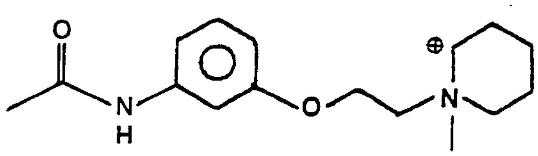 | Br$^{\ominus}$ | Fp. 174-178°C |
| 106. | 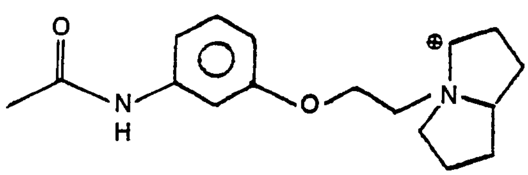 | Br$^{\ominus}$ | Harz |
| 107. | 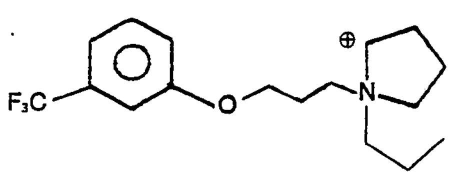 | Br$^{\ominus}$ | Fp. 103°C |

| | | | |
|---|---|---|---|
| 108. | 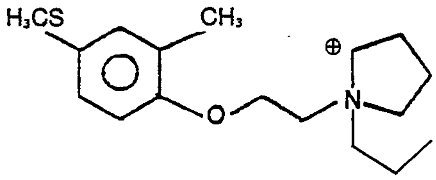 | Br$^{\ominus}$ | Fp. 85-100°C |
| 109. | 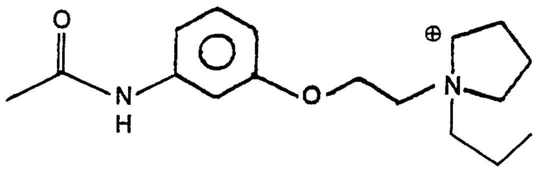 | Br$^{\ominus}$ | Harz |
| 110. | 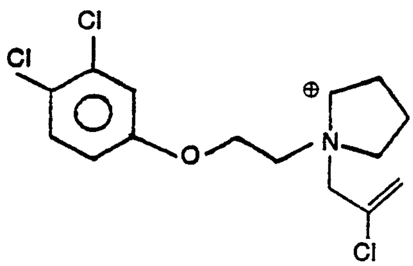 | Cl$^{\ominus}$ | Fp. 126-130°C |
| 111. | 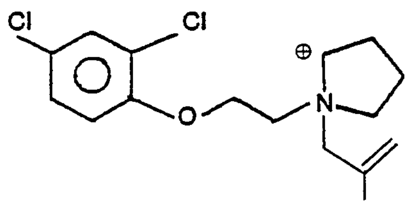 | Cl$^{\ominus}$ | Fp. 85-89°C |
| 112. | 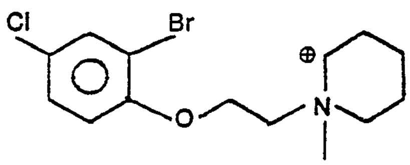 | Br$^{\ominus}$ | Fp. 166-168°C |
| 113. | 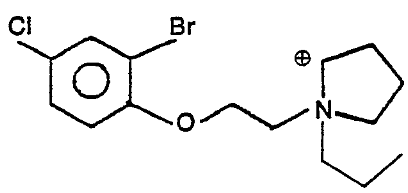 | Br$^{\ominus}$ | Fp. 149-155°C |
| 114. | 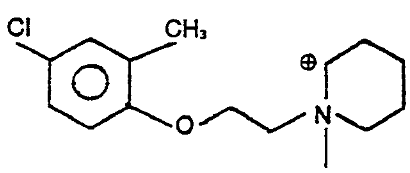 | Br$^{\ominus}$ | Fp. 64-68°C |

115. 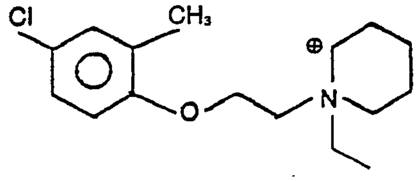 $Br^{\ominus}$ Fp. 96-99°C

116. 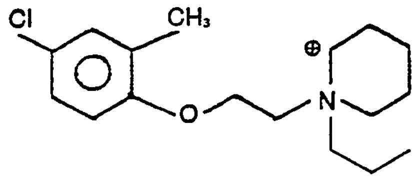 $Br^{\ominus}$ Fp. 149-150°C

117. 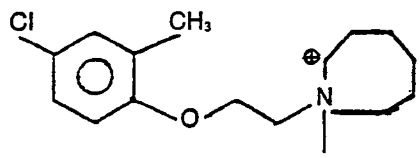 $Br^{\ominus}$ Fp. 124-127°C

118. 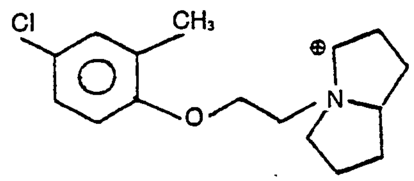 $Br^{\ominus}$ Fp. 139-148°C

119. 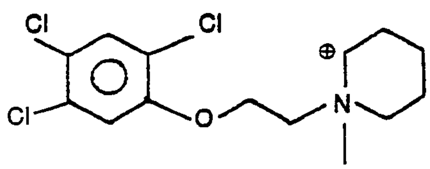 $Br^{\ominus}$ Fp. 96-99°C

120. 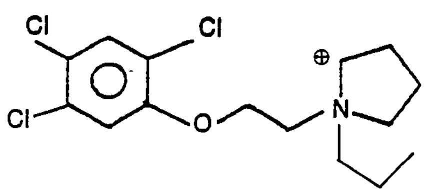 $Br^{\ominus}$ Fp. 160-164°C

121. 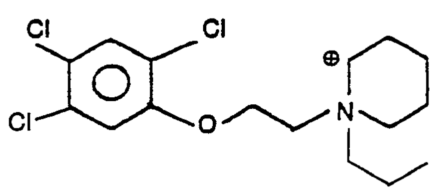 $Br^{\ominus}$ Fp. 180-184°C

122. 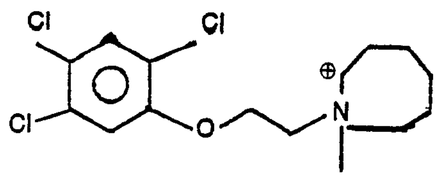 Br⊖ Fp. 90-92°C

123. 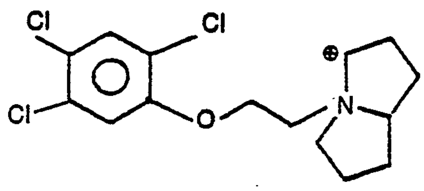 Br⊖ Fp. 159-160°C

124. 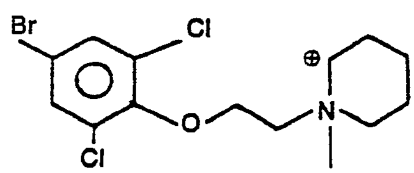 Br⊖ Fp. 225-227°C

125. 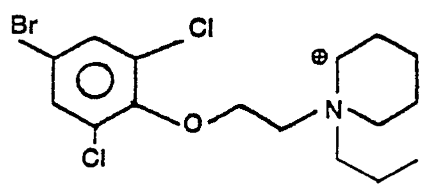 Br⊖ Fp. 180-184°C

126. 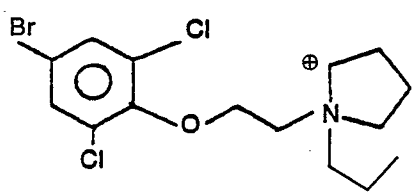 Br⊖ Fp. 181-184°C

127. 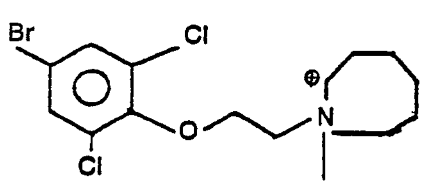 Br⊖ Fp. >230°C

128. 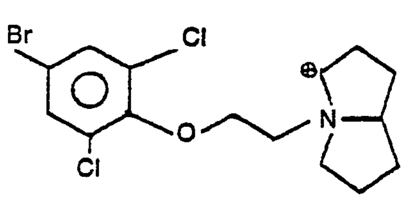 Br⊖ Fp. 90-92°C

129. 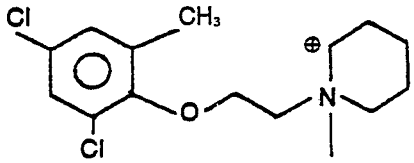 Br$^{\ominus}$ Fp. 193-197°C

130. 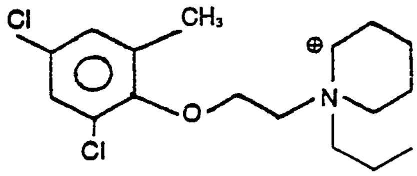 Br$^{\ominus}$ Fp. 141-147°C

131. 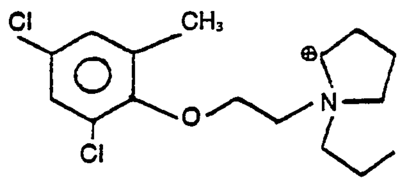 Br$^{\ominus}$ Fp. 69-73°C

132. 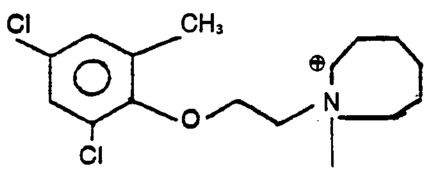 Br$^{\ominus}$ Fp. 225-227°C

133. 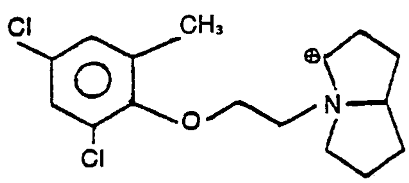 Br$^{\ominus}$ Fp. 90-95°C

134. 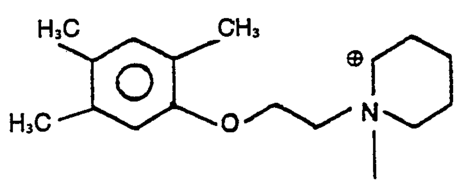 Br$^{\ominus}$ Fp. 152-155°C

135. 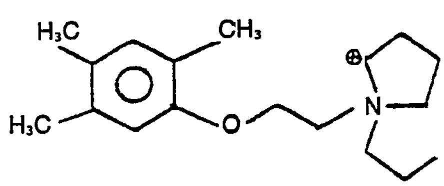 Br$^{\ominus}$ Fp. 182-184°C

136.

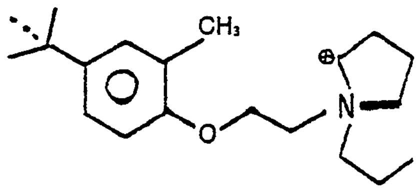

$Br^{\ominus}$

137.

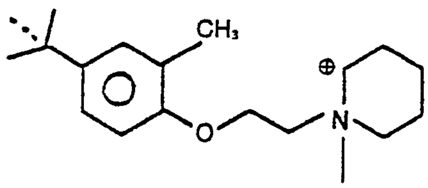

$Br^{\ominus}$

138.

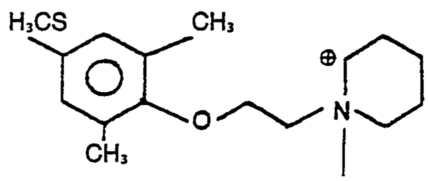

$Br^{\ominus}$

139.

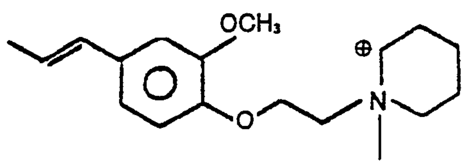

$Br^{\ominus}$

140.

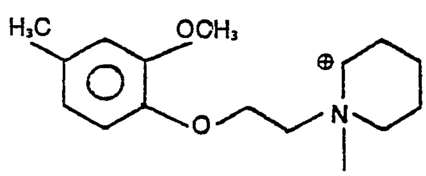

$Br^{\ominus}$

141.

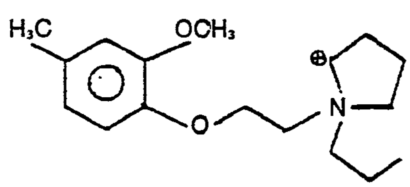

$Br^{\ominus}$

142.

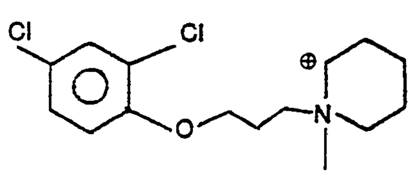

$Br^{\ominus}$

143. 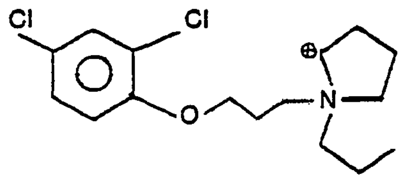

$Br^{\ominus}$

144. 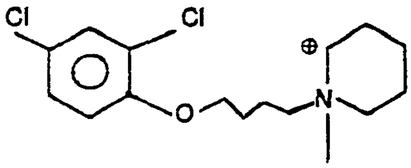

$Br^{\ominus}$

145. 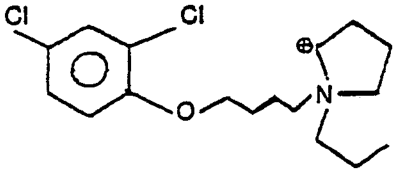

$Br^{\ominus}$

Die neuen erfindungsgemässen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach den bisherigen Erfahrungen, dass ein Wirkstoff eine oder auch mehrere verschiedenartige Wirkungen auf Pflanzen ausüben kann.

Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren (Samenbeize, Bodenbehandlung oder Blattapplikation),

d) von den geoklimatischen Faktoren, z. B. Sonnenscheindauer, Durchschnittstemperatur, Niederschlagsmenge, von der Bodenbeschaffenheit (einschliesslich Düngung),

f) von der Formulierung bzw. Anwendungsform des Wirkstoffs und schliesslich

g) von den angewendeten Konzentrationen der aktiven Substanz.

In jedem Falle sollen Wachstumsregulatoren die Kulturpflanzen in gewünschter Weise positiv beeinflussen.

Aus der Reihe der verschiedenartigen Anwendungsmöglichkeiten der erfindungsgemässen Pflanzenwachstumsregulatoren im Pflanzenanbau, in der Landwirtschaft und im Gartenbau, werden einige nachstehend erwähnt.

A. Mit den erfindungsgemäss verwendbaren Verbindungen lässt sich das vegetative Wachstum der Pflanzen stark hemmen, was sich insbesondere in einer Reduzierung des Längenwachstums äussert. Die behandelten Pflanzen weisen demgemäss einen gedrungenen Wuchs auf. Parallel hierzu führt die Anwendung der erfindungsgemässen Verbindungen zu einem erhöhten Chlorophyllgehalt und damit zu einer dunkleren Blattfärbung. Infolgedessen lässt sich eine erhöhte Photosyntheserate und damit eine erhöhte Ertragsbildung erwarten.

Als vorteilhaft für die Praxis erweist sich z. B. die Verringerung des Grasbewuchses an Strassenrändern, Kanalböschungen und auf Rasenflächen wie Park-, Sport- und Ostanlagen, Zierrasen und Flugplätzen, so dass der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des «Lagerns» (des Umknickens) von Pflanzen unter ungünstigen Witterungsbedingungen von der Ernte.

Wichtig ist auch die Anwendung von Wachstumsregulatoren zum Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung von Wachstumsregulatoren kann auch die seitliche Verzweigung der Pflanzen vermehrt oder gehemmt werden. Daran besteht Interesse, wenn z. B. bei Tabakpflanzen die Ausbildung von Seitentrieben (Geiztrieben) zugunsten des Blattwachstums gehemmt werden soll.

Ein weiterer Mechanismus der Ertragssteigerung mit Wachstumshemmern beruht darauf,

dass die Nährstoffe in stärkerem Masse der Blüten- und Fruchtbildung zugute kommen, während das vegetative Wachstum eingeschränkt wird. Ferner kann so wegen der relativ geringen Blatt- bzw. Pflanzenmasse dem Befall mit verschiedenen, insbesondere pilzlichen Krankheiten vorgebeugt werden.

Die Hemmung des vegetativen Wachstums ermöglicht ausserdem bei vielen Kulturpflanzen eine dichtere Bepflanzung, so dass ein Mehrertrag bezogen auf die Bodenfläche erzielt werden kann. Die erfindungsgemäsen Verbindungen eignen sich besonders zur Hemmung des vegetativen Wachstums bei Kulturpflanzen wie Soja, Sonnenblumen, Erdnüssen, Raps, Zierpflanzen, Baumwolle, Reis und Gräsern.

B. Mit den neuen Wirkstoffen lassen sich Mehrerträge sowohl an Pflanzenteilen als auch an Pflanzeninhaltsstoffen erzielen. So ist es beispielsweise möglich, das Wachstum grösserer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluss zu stimulieren.

Dabei können die neuen Stoffe Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schliesslich sowohl eine Verkürzung bzw. Verlängerung der Wachstumsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, dass heisst die Förderung der Ausbildung von Trenngewebe zwischen Frucht- bzw. Blatt und Sprossteil der Pflanze ist auch für ein gut kontrollierbares Entblättern der Bäume wesentlich.

Die Wirkung der neuen Verbindungen ist besser als bei bekannten Wachstumsregulatoren. Die Wirkung zeigt sich sowohl bei Monokotylen, z. B. Getreide wie Weizen, Gerste, Roggen, Hafer und Reis oder Mais oder Gräsern als auch insbesondere bei Dikotylen (z. B. Sonnenblumen, Tomaten, Erdnüssen, Reben, Baumwolle, Raps und vor allem Soja) und verschiedenen Zierpflanzen wie Chrysanthemen, Poinsettien und Hibiskus.

Die erfindungsgemässen Wirkstoffe können den Kulturpflanzen sowohl vom Samen her (als Saatgutbeizmittel) als auch über den Boden, d. h. durch die Wurzel sowie — besonders bevorzugt — durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g, benötigt.

Für die Blatt- und Bodenbehandlung sind im allgemeinen Gaben von 0,001 bis 12 kg/ha bevorzugt 0,01 bis 3 kg/ha als ausreichend zu betrachten.

Die erfindungsgemässen Mittel können in Form üblicher Formulierungen angewendet werden wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmässige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel zugesetzt werden können. Als Hilfsstoffe zur Formulierung kommen im wesentlichen in Frage Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Amine (z. B. Ethanolamin), Ketone (z. B. Cyclohexanon), Dimethylformamid und Wasser; feste Trägerstoffe wie natürliche Gesteinsmehle (z. B. Kaoline, Tonerden, Talkum, Kreide) und synthetische Gesteinsmehle (z. B. hochdisperse Kieselsäure, Silikate); Emulgiermittel oder sonstige oberflächenaktive Mittel, wie nichtionogene und anionische Emulgatoren (z. B. Polyoxyethylen-Fettalkohol-Ether, Alkylsulfonate) und Dispergiermittel wie Lignin, Sulfitablaugen und Methylcellulose. Bevorzugt ist die Anwendung der erfindungsgemässen Verbindungen in wässriger Lösung gegebenenfalls unter Zusatz von mit Wasser mischbaren organischen Lösungsmitteln wie Methanol oder anderen niederen Alkoholen, Aceton, Dimethylformamid oder N-Methylpyrrolidin. Die Formulierungen enthalten im allgemeinen zwischen 0,1 und 95 Gewichtsprozent Wirkstoff, vorzugsweise zwischen 0,5 und 90.

Die Formulierungen bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise im Vorauflaufverfahren, im Nachauflaufverfahren oder als Beizmittel.

Beispiele für Formulierungen sind:

I 20 Gewichtsteile der Verbindung des Beispiels 10 werden in 3 Gewichtsteilen des Natriumsalzes der Diisobutylnaphthalin-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.

II 3 Gewichtsteile der Verbindung des Beispiels 15 werden mit 97 Gewichtsteilen feinteiligem Kaolin innig vermischt. Man erhält auf diese Weise ein Stäubemittel, das 3 Gew.-% des Wirkstoffs enthält.

III 30 Gewichtsteile der Verbindung des Beispiels 18 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV 40 Gewichtsteile der Verbindung des Beispiels 20 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wässrige Dispersion. Durch Verdünnen mit 100 000 Gewichtsteilen Wasser erhält man eine wässrige Dispersion, die 0,04 Gew.-% Wirkstoff enthält.

V 20 Teile der Verbindung des Beispiels 29 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

VI Man vermischt 90 Gewichtsteile der Verbindung des Beispiels 27 mit 10 Gewichtsteilen N-Methyl-α-pyrrolidon und erhält eine Lösung, die zur Anwendung in Form kleinster Tropfen geeignet ist.

VII 20 Gewichtsteile der Verbindung des Beispiels 28 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgiessen und feines Verteilen der Lösung in 100 000 Gewichtsteilen Wasser erhält man eine wässrige Dispersion, die 0,02 Gew.-% des Wirkstoffs enthält.

VIII 20 Gewichtsteile der Verbindung des Beispiels 30 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinuslöl besteht. Durch Eingiessen und feines Verteilen der Lösung in 100 000 Gewichtsteilen Wasser erhält man eine wässrige Dispersion, die 0,02 Gew.-% des Wirkstoffs enthält.

IX 10 Gewichtsteile der Verbindung des Beispiels 10, 20 Gewichtsteile Polyoxyethylensorbitan-monolaurat (Tween 20®), 20 Gewichtsteile Methanol und 50 Gewichtsteile Wasser werden zu einer Lösung verrührt, die 10 Gew.-% des Wirkstoffs enthält. Durch Hinzufügen von weiterem Wasser können stärker verdünnte Lösungen hergestellt werden.

Die erfindungsgemässen Mittel können in diesen Aufwendungsformen auch zusammen mit anderen Wirkstoffen vorliegen, wie z. B. Herbiziden, Insektiziden, Wachstumsregulatoren und Fungiziden oder auch mit Düngemitteln vermischt und ausgebracht werden. Bei Vermischen mit Wachstumsregulatoren erhält man dabei in vielen Fällen eine Vergrösserung des Wirkungsspektrums. Bei einer Anzahl solcher Wachstumsregulatormischungen treten auch synergistische Effekte auf d. h. die Wirksamkeit des Kombinationsproduktes ist grösser als die addierten Wirksamkeiten der Einzelkomponenten.

Fungizide, die mit den erfindungsgemässen Verbindungen kombiniert werden können, sind beispielsweise Dithiocarbamate und deren Derivate, wie
Ferridimethyldithiocarbamat,
Zinkdimethyldithiocarbamat,
Manganethylenbisthiocarbamat,
Mangan-Zink-ethylendiamin-bis-dithiocarbamat,
Zinkethylenbisthiocarbamat,
Tetramethylthiuramdisulfide,
Ammoniak-Komplex von Zink-(N,N-ethylen-bis--dithiocarbamat) und
N,N'-Polyethylen-bis-(thiocarbamoyl)-disulfid,
Zink-(N,N'-propylen-bis-dithiocarbamat),
Ammoniak-Komplex von Zinn-(N,N'-propylen-bis--dithiocarbamat) und
N,N'-Polypropylen-bis-(thiocarbamoyl)-disulfid;

Nitrophenolderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;

heterocyclische Verbindung, wie
N-Trichlormethylthio-tetrahydrophthalimid,
N-Trichlormethylthio-phthalimid,
2-Heptadecyl-2-imidazol-acetat,
2,4-Dichlor-6-(o-chloranilino)-s-triazin,
O,O-Diethyl-phthalimidophosphonthionat,
5-Amino-1-[bis-(dimethylamino)-phosphinyl]-3--phenyl-1,2,4-triazol,
5-Ethoxy-3-trichlormethyl-1,2,4-thiadiazol,
2,3-Dicyano-1,4-dithiaanthrachinon,
2-Thio-1,3-dithio-(4,5-b)-chinoxalin,
1-Butylcarbamoyl-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thiol-1-oxid,
8-Hydrochinolin bzw. dessen Kupfersalz,
2,3-Dihydro-5-carboxanilido-6-methyl-1,4-oxathiin-4,4-dioxid,

2,3-Dihydro-5-carboxanilido-6-methyl-1,4-oxathiin,
2-(Furyl-2)-benzimidazol,
Piperazin-1,4-diyl-bis-1-(2,2,2-trichlor-ethyl)-formamid,
2-Thiazolyl-(4)-benzimidazol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

und verschiedene Fungizide, wie
Dodecylguanidinacetat,
3-[2-(3,5-Dimethyl-2-oxycyclohexyl)-2-hydroxyethyl]-glutarimid,
Hexachlorbenzol,
N-Dichlorfluormethylthio-N,N'-dimethyl-N-phenyl-schwefelsäurediamid,
D,L-Methyl-N-(2,6-dimethyl-phenyl)-N-furyl-(2)-alaninat,
D,L-N-(2,6-Dimethyl-phenyl)-N-(2'-methoxyacetyl)-alanin-methylester,
5-Nitro-isophthalsäure-di-isopropylester,
2,5-Dimethyl-furan-3-carbonsäureanilid,
2,5-Dimethyl-furan-3-carbonsäure-cyclohexylamid,
2-Methyl-benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,
2,3-Dichlor-1,4-naphthochinon,
1,4-Dichlor-2,5-dimethoxybenzol,
p-Dimethylaminobenzol-diazinnatriumsulfonat,
1-Chlor-2-nitro-propan,

Polychlornitrobenzole, wie Pentachlornitrobenzol, Methylisocyanat, fungizide Antibiotika, wie Griseofulvin oder Kasugamycin, Tetrafluordichloraceton, 1-Phenylthiosemicarbazid, Bordeauxmischung, nickelhaltige Verbindungen und Schwefel.

In den nachfolgenden Beispielen A und B wird die Wirkung der erfindungsgemäss verwendbaren Stoffe als Pflanzenwachstumsregulatoren dargestellt, ohne die Möglichkeit weiterer Anwendungen als Wachstumsregulatoren auszuschliessen.

Beispiel A, Gewächshausversuche

Zur Bestimmung der wachstumsregulierenden Eigenschaft der Prüfsubstanzen wurden Testpflanzen auf ausreichend mit Nährstoffen versorgtem Torfkultursubstrat in Kunststoffgefässen von ca. 12,5 cm Durchmesser angezogen.

Im Nachauflaufverfahren wurden die zu prüfenden Substanzen in wässriger Aufarbeitung auf die Pflanzen gesprüht. Die beobachtete wachstumsregulierende Wirkung wurde bei Versuchsende durch Wuchshöhenmessung belegt. Die so gewonnenen Messwerte wurden zur Wuchshöhe der unbehandelten Pflanzen in Relation gesetzt. Als Vergleichssubstanz diente CCC:

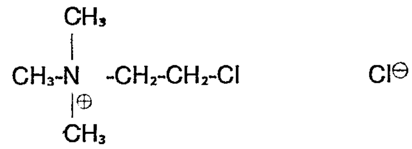

und die aus der DE-OS 20 17 497 bekannte Substanz

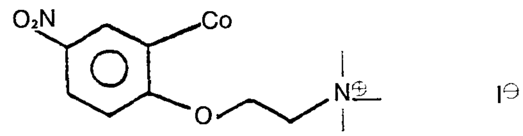 (Z).

Gleichlaufend zur Reduzierung des Längenwachstums stieg die Farbintensität der Blätter an. Der erhöhte Chlorophyllgehalt spricht für eine erhöhte Photosyntheserate und damit für eine erhöhte Ertragsbildung.

Die Einzeldaten sind den in den Tabellen 1 bis 4 wiedergegeben Versuchsreihen zu entnehmen.

Beispiel B, Vegetationsversuch

In einem Vegetationsversuch wurden Sojabohnen der Sorte SRF 400 auf einem neutralen, lehmigen Sandboden, der ausreichend mit Nährstoffen versorgt wurde, in Mitscherlichgefässer unter optimalen Bedingungen in einer Klimakammer angebaut. Die Applikation der Prüfsubstanzen erfolgte im Nachauflaufverfahren durch Besprühen der Pflanzen mit wässrigen Aufbereitungen der Substanzen.

Die angewandten Aufwandmengen entsprachen 0,5, 1,0 und 1,5 kg Wirkstoff/ha. Jeweils 2 Gefässe bildeten eine Versuchsvariante. Bei Versuchsende wurde die beobachtete einkürzende Wirkung durch Wuchshöhenmessung belegt. Diese Messwerte wurden zur Wuchshöhe der unbehandelten Pflanzen in Relation gesetzt.

Als Vergleichssubstanz diente CCC und die Substanz Z (vgl. DE-OS 20 17 497):

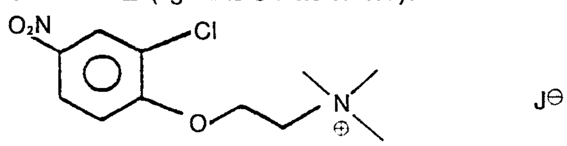

Die Einzeldaten sind Tabelle 5 zu entnehmen.

TABELLE 1

Einfluss auf das Längenwachstum von Monokotylen
Rasen, Nachauflaufverfahren;
Versuchsdauer: 45 Tage

| Substanz Nr. | Konz. mg WS/Gefäss | Wuchshöhen cm | % |
|---|---|---|---|
| unbehandelt | — | 9,1 | 100 |
| CCC | 6 | 8,5 | 93,4 |
| 10 | 6 | 7,5 | 82,4 |

TABELLE 2.1

Einfluss auf das Längenwachstum von Dikotylen
Gewächshausversuche
Sojabohnen, Sorte SRF 400
Nachauflaufverfahren; Versuchsdauer: 31 Tage

| Substanz Nr. | Konz. mg WS/Gefäss | Wuchshöhen cm | % |
|---|---|---|---|
| unbehandelt | — | 25,3 | 100 |
| CCC | 1,5 | 24,5 | 96,8 |
| | 6,0 | 23,5 | 92,9 |
| 10 | 1,5 | 15,0 | 59,3 |
| | 6,0 | 13,5 | 53,4 |
| 15 | 1,5 | 21,5 | 85,0 |
| | 6,0 | 15,0 | 59,3 |
| 16 | 1,5 | 16,0 | 63,2 |
| | 6,0 | 13,5 | 53,4 |
| 18 | 1,5 | 17,0 | 67,2 |
| | 6,0 | 15,0 | 59,3 |
| 26 | 1,5 | 18,0 | 71,2 |
| | 6,0 | 16,5 | 65,2 |
| 27 | 1,5 | 16,5 | 65,2 |
| | 6,0 | 14,5 | 57,3 |
| 28 | 1,5 | 15,0 | 59,3 |
| | 6,0 | 14,0 | 55,3 |
| 30 | 1,5 | 17,0 | 67,2 |
| | 6,0 | 13,0 | 51,4 |

(WS = Wirkstoff)

TABELLE 2.2

Einfluss auf das Längenwachstum von Dikotylen
Gewächshausversuche
Sojabohnen, Sorte SRF 400
Nachauflaufverfahren; Versuchsdauer: 31 Tage

| Substanz Nr. | Konz. mg WS/Gefäss | Wuchshöhen % |
|---|---|---|
| unbehandelt | — | 100 |
| CCC | 1,5 | 92,5 |
| | 6,0 | 83,0 |
| Z | 1,5 | 94,3 |
| | 6,0 | 92,5 |
| 5 | 1,5 | 84,9 |
| | 6,0 | 73,6 |
| 7 | 1,5 | 79,2 |
| | 6,0 | 71,7 |
| 8 | 1,5 | 43,4 |
| | 6,0 | 37,7 |
| 13 | 1,5 | 47,2 |
| | 6,0 | 41,5 |
| 21 | 1,5 | 49,1 |
| | 6,0 | 34,0 |
| 22 | 1,5 | 52,8 |
| | 6,0 | 45,3 |
| 23 | 1,5 | 67,9 |
| | 6,0 | 58,5 |
| 24 | 1,5 | 50,0 |
| | 6,0 | 52,1 |

TABELLE 2.3

Einfluss auf das Längenwachstum von Dikotylen
Gewächshausversuche
Sojabohnen, Sorte SRF 400
Nachauflaufverfahren; Versuchsdauer: 31 Tage

| Substanz Nr. | Konz. mg WS/Gefäss | Wuchshöhen % |
|---|---|---|
| unbehandelt | — | 100 |
| CCC | 1,5 | 88,4 |
| | 6,0 | 79,0 |
| Z | 1,5 | 94,0 |
| | 6,0 | 94,0 |
| 36 | 1,5 | 94,0 |
| | 6,0 | 82,7 |
| 52 | 1,5 | 94,0 |
| | 6,0 | 82,7 |
| 62 | 1,5 | 77,1 |
| | 6,0 | 56,4 |

TABELLE 2.4

Einfluss auf das Längenwachstum von Dikotylen
Gewächshausversuche
Sojabohnen, Sorte SRF 400
Nachauflaufverfahren; Versuchsdauer: 31 Tage

| Substanz Nr. | Konz. mg WS/Gefäss | Wuchshöhen % |
|---|---|---|
| unbehandelt | — | 100 |
| CCC | 1,5 | 85,6 |
| | 6,0 | 85,6 |
| 40 | 1,5 | 48,0 |
| | 6,0 | 41,1 |
| 41 | 1,5 | 41,1 |
| | 6,0 | 37,7 |
| 43 | 1,5 | 54,8 |
| | 6,0 | 41,1 |

TABELLE 2.5

Einfluss auf das Längenwachstum von Dikotylen
Gewächshausversuche
Sojabohnen, Sorte SRF 400
Nachauflaufverfahren; Versuchsdauer: 31 Tage

| Substanz Nr. | Konz. mg WS/Gefäss | Wuchshöhen % |
|---|---|---|
| unbehandelt | — | 100 |
| CCC | 1,5 | 98,0 |
| | 6,0 | 91,4 |
| 32 | 1,5 | 79,7 |
| | 6,0 | 63,1 |
| 37 | 1,5 | 59,8 |
| | 6,0 | 53,2 |
| 42 | 1,5 | 38,2 |
| | 6,0 | 34,9 |
| 53 | 1,5 | 66,5 |
| | 6,0 | 59,8 |
| 57 | 1,5 | 76,4 |
| | 6,0 | 56,5 |
| 59 | 1,5 | 53,2 |
| | 6,0 | 46,5 |
| 31 | 1,5 | 49,8 |
| | 6,0 | 43,2 |
| 84 | 1,5 | 89,7 |
| | 6,0 | 89,7 |
| 101 | 1,5 | 53,2 |
| | 6,0 | 43,2 |
| 107 | 1,5 | 49,8 |
| | 6,0 | 41,5 |

TABELLE 2.6

Einfluss auf das Längenwachstum von Dikotylen
Gewächshausversuche
Sojabonen, Sorte SRF 400
Nachauflaufverfahren; Versuchsdauer: 31 Tage

| Substanz Nr. | Konz. mg WS/Gefäss | Wuchshöhen % |
|---|---|---|
| unbehandelt | — | 100 |
| CCC | 1,5 | 90,0 |
| | 6,0 | 90,0 |
| 34 | 1,5 | 83,0 |
| | 6,0 | 58,8 |
| 86 | 1,5 | 86,5 |
| | 6,0 | 83,0 |
| 97 | 1,5 | 93,4 |
| | 6,0 | 83,0 |

TABELLE 2.7

Einfluss auf das Längenwachstum von Dikotylen
Gewächshausversuche
Sojabohnen, Sorte SRF 400
Nachauflaufverfahren; Versuchsdauer: 31 Tage

| Substanz Nr. | Konz. mg WS/Gefäss | Wuchshöhen % |
|---|---|---|
| unbehandelt | — | 100 |
| CCC | 1,5 | 89,2 |
| | 6,0 | 83,3 |
| 6 | 1,5 | 73,2 |
| | 6,0 | 67,1 |
| 12 | 1,5 | 61,0 |
| | 6,0 | 48,8 |
| 14 | 1,5 | 67,1 |
| | 6,0 | 48,8 |
| 47 | 1,5 | 77,2 |
| | 6,0 | 77,2 |
| 64 | 1,5 | 81,3 |
| | 6,0 | 75,2 |
| 100 | 1,5 | 67,1 |
| | 6,0 | 63,0 |

TABELLE 2.8

Einfluss auf das Längenwachstum von Dikotylen
Gewächshausversuche
Sojabohnen, Sorte SRF 400
Nachauflaufverfahren; Versuchsdauer: 31 Tage

| Substanz Nr. | Konz. mg WS/Gefäss | Wuchshöhen % |
|---|---|---|
| unbehandelt | — | 100 |
| CCC | 1,5 | 90,9 |
| | 6,0 | 77,9 |
| 38 | 1,5 | 65,0 |
| | 6,0 | 47,1 |
| 96 | 1,5 | 50,3 |
| | 6,0 | 40,3 |
| 105 | 1,5 | 69,6 |
| | 6,0 | 50,3 |
| 106 | 1,5 | 71,4 |
| | 6,0 | 53,6 |
| 109 | 1,5 | 55,2 |
| | 6,0 | 52,0 |
| 110 | 1,5 | 61,7 |
| | 6,0 | 52,0 |
| 111 | 1,5 | 56,8 |
| | 6,0 | 52,0 |
| 112 | 1,5 | 64,9 |
| | 6,0 | 53,6 |

TABELLE 2.9

Einfluss auf das Längenwachstum von Dikotylen
Gewächshausversuche
Sojabohnen, Sorte SRF 400
Nachauflaufverfahren; Versuchsdauer: 31 Tage

| Substanz Nr. | Konz. mg WS/Gefäss | Wuchshöhen % |
|---|---|---|
| unbehandelt | — | 100 |
| CCC | 1,5 | 91,3 |
| | 6,0 | 76,1 |
| 120 | 1,5 | 91,3 |
| | 6,0 | 76,1 |
| 121 | 1,5 | 87,5 |
| | 6,0 | 76,1 |
| 122 | 1,5 | 87,5 |
| | 6,0 | 87,5 |
| 123 | 1,5 | 87,5 |
| | 6,0 | 83,7 |
| 125 | 1,5 | 72,2 |
| | 6,0 | 64,6 |
| 133 | 1,5 | 68,4 |
| | 6,0 | 64,6 |

TABELLE 3

Einfluss auf das Längenwachstum von Dikotylen
Gewächshausversuche
Sommerraps, Sorte Cosa
Nachauflaufverfahren; Versuchsdauer: 25 Tage

| Substanz Nr. | Konz. mg WS/Gefäss | Wuchshöhen cm | % |
|---|---|---|---|
| unbehandelt | — | 25,5 | 100 |
| CCC | 1,5 | 23,0 | 90,2 |
| | 6,0 | 21,5 | 84,3 |
| 18 | 1,5 | 20,0 | 78,4 |
| | 6,0 | 15,0 | 58,8 |
| 26 | 1,5 | 22,5 | 88,2 |
| | 6,0 | 20,0 | 78,4 |
| 27 | 1,5 | 22,5 | 88,2 |
| | 6,0 | 17,0 | 66,7 |
| 28 | 1,5 | 22,0 | 86,3 |
| | 6,0 | 18,0 | 70,6 |
| 30 | 1,5 | 23,0 | 90,2 |
| | 6,0 | 18,0 | 70,6 |

TABELLE 4

Einfluss auf das Längenwachstum von Dikotylen
Gewächshausversuche
Sonnenblumen
Nachauflaufverfahren; Versuchsdauer: 21 Tage

| Substanz Nr. | Konz. mg WS/Gefäss | Wuchshöhen cm | % |
|---|---|---|---|
| unbehandelt | — | 28,5 | 100 |
| CCC | 1,5 | 26,0 | 91,2 |
| | 6,0 | 24,0 | 84,2 |
| 10 | 1,5 | 25,0 | 87,7 |
| | 6,0 | 20,0 | 70,2 |
| 15 | 1,5 | 23,0 | 80,7 |
| | 6,0 | 18,0 | 63,2 |
| 16 | 1,5 | 26,0 | 91,2 |
| | 6,0 | 20,0 | 70,2 |
| 18 | 1,5 | 22,0 | 77,2 |
| | 6,0 | 21,0 | 73,7 |
| 26 | 1,5 | 19,0 | 66,7 |
| | 6,0 | 15,0 | 52,6 |
| 27 | 1,5 | 18,0 | 63,2 |
| | 6,0 | 15,0 | 52,6 |
| 28 | 1,5 | 18,0 | 63,2 |
| | 6,0 | 16,0 | 56,1 |
| 30 | 1,5 | 20,0 | 70,2 |
| | 6,0 | 16,0 | 56,1 |

TABELLE 5

Vegetationsversuch in der Klimakammer
Sojabohnen, Sorte SRF 400, Nachauflaufverfahren; Versuchsdauer: 69 Tage

| Substanz Nr. | Konz. mg WS/Gefäss | Wuchshöhen cm | % |
|---|---|---|---|
| unbehandelt | — | 98,8 | 100 |
| CCC | 0,5 | 96,0 | 97,2 |
| | 1,0 | 94,5 | 95,7 |
| | 1,5 | 91,0 | 92,1 |
| Z | 0,5 | 97,5 | 98,7 |
| | 1,0 | 95,0 | 96,2 |
| | 1,5 | 95,0 | 96,2 |
| 10 | 0,5 | 82,5 | 83,5 |
| | 1,0 | 76,5 | 77,4 |
| | 1,5 | 64,0 | 64,8 |
| 28 | 0,5 | 86,5 | 87,6 |
| | 1,0 | 78,5 | 79,5 |
| | 1,5 | 73,5 | 74,4 |

**Patentansprüche**
für die Vertragsstaaten BE, CH, DE, FR, GB, IT, LU, NL, SE

1. Substituierte Alkylammoniumsalze der Formel I

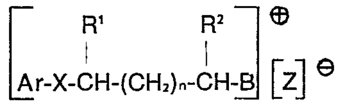 (I)

in der

Ar Phenyl bedeutet, das durch eine Trifluoromethyl-, Nitro-, Cyano-, $C_2$-$C_4$-Alkylcarbonyl-, $C_2$-$C_4$-Alkylcarbonylaminogruppe oder 2 bis 3 gleiche oder verschiedene der folgenden Substituenten substituiert ist: Alkyl, Alkenyl, Alkinyl, Cycloalkyl, Alkoxy, Alkenyloxy, Alkinyloxy, Alkylthio, Halogenalkyl, Halogenalkoxy, Halogenalkylthio, Alkylcarbonylamino, Alkylsulfonyl, Alkylaminosulfonyl, Dialkylaminosulfonyl mit jeweils bis zu 6 C-Atomen, Fluor, Chlor, Brom, Jod, Nitro, Cyano, Aminosulfonyl, Phenyl oder Benzyl,

X Sauerstoff oder Schwefel darstellt,

n die Zahl 0, 1 oder 2 ist,

$R^1$ und $R^2$ gleich oder verschieden sind und Wasserstoff oder $C_1$-$C_4$-Alkyl bedeuten,

B einen Chinuclidin- oder Pyrrolizidinbicyclus oder die Gruppe 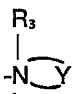 darstellt, in welcher

Y für eine gegebenenfalls durch 1 bis 3 gleiche oder verschiedene $C_1$-$C_4$-Alkyl-, -Alkoxy-, -Halogenalkyl-, Chlor-, Brom-, Hydroxy- oder Cyanoreste substituierte $-(CH_2)_4-$, $-(CH_2)_5-$, $-(CH_2)_6-$, $-(CH_2)_7-$, $-CH_2-CH=CH-(CH_2)_2-$, $-(CH_2)_2-O-(CH_2)_2-$, $-(CH_2)_2-S-(CH_2)_2-$, $-NH-(CH_2)_4-$ oder $-NH-CH_2-CH=CH-CH_2-$Gruppe steht und

$R^3$ geradkettiges oder verzweigtes $C_1$-$C_6$-Alkyl, -Alkenyl oder -Alkinyl bedeutet, das durch Halogen, Hydroxy, Cyano, $C_1$-$C_4$-Alkoxy oder -Alkylcarbonyl substituiert sein kann und

Z das Anion einer beliebigen, nicht phytotoxischen Säure HX bedeutet.

2. Verfahren zur Herstellung substituierter Alkylammoniumsalze der Formel I gemäss Anspruch 1, dadurch gekennzeichnet, dass man in an sich bekannter Weise

a) Verbindungen der Formel II

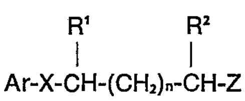 (II)

in der Ar, X, $R^1$, n und $R^2$ die oben angegebene Bedeutung haben und Z für eine nucleofuge Abgangsgruppe steht, mit einem tertiären Amin der Formel III

B (III)

in der B die oben angegebenen Bedeutungen hat, umsetzt oder

b) Verbindungen der Formel IV

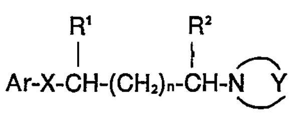 (IV)

in der Ar, X, $R^1$, n, $R^2$ und Y die oben genannten Bedeutungen haben, mit Alkylierungsmitteln der Formel V

$R^3$-Z (V)

in der $R^3$ die oben angegebene Bedeutung hat und Z für eine nucleofuge Abgangsgruppe steht, zum Quartärsalz der Formel I alkyliert.

3. Mittel zur Regulierung des Pflanzenwachstums, enthaltend eine oder mehrere Verbindungen gemäss Anspruch 1.

4. Mittel zur Regulierung des Pflanzenwachstums, enthaltend eine oder mehrere Verbindungen gemäss Anspruch 1 und einen flüssigen oder festen Trägerstoff sowie gegebenenfalls eine oder mehrere oberflächenaktive Mittel.

5. Verwendung von Verbindungen gemäss Anspruch 1 zur Regulierung des Pflanzenwachstums.

6. Verfahren zur Herstellung von das Pflanzenwachstum regulierenden Mitteln, dadurch gekennzeichnet, dass man eine oder mehrere Verbindungen gemäss Anspruch 1 mit festen oder flüssigen Trägerstoffen sowie gegebenenfalls einem oder mehreren oberflächenaktiven Mitteln mischt.

**Patentansprüche**

für den Vertragsstaat AT

1. Mittel zur Regulierung des Pflanzenwachstums enthaltend ein substituiertes Alkylammoniumsalz der Formel I

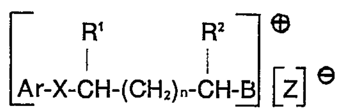 (I)

in der

Ar Phenyl bedeutet, das durch eine Trifluoromethyl-, Nitro-, Cyano-, $C_2$-$C_4$-Alkylcarbonyl, $C_2$-$C_4$-Alkylcarbonylaminogruppe oder durch 2 bis 3 gleiche oder verschiedene der folgenden Substituenten substituiert ist: Alkyl, Alkenyl, Alkinyl, Cycloalkyl, Alkoxy, Alkenyloxy, Alkinyloxy, Alkylthio, Halogenalkyl, Halogenalkoxy, Halogenalkylthio, Alkylcarbonylamino, Alkylsulfonyl, Alkylaminosulfonyl, Dialkylaminosulfonyl mit jeweils bis zu 6 C-Atomen, Fluor, Chlor, Brom, Jod, Nitro, Cyano, Aminosulfonyl, Phenyl oder Benzyl,

X Sauerstoff oder Schwefel darstellt,

n die Zahl 0, 1 oder 2 ist,

$R^1$ und $R^2$ gleich oder verschieden sind und Wasserstoff oder $C_1$-$C_4$-Alkyl bedeuten,

B einen Chinuclidin- oder Pyrrolizidinbicyclus oder die Gruppe 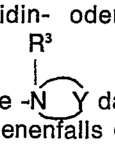 darstellt, in welcher

Y für eine gegebenenfalls durch 1 bis 3 gleiche oder verschiedene $C_1$-$C_4$-Alkyl-, -Alkoxy-, -Halogenalkyl-, Chlor-, Brom-, Hydroxy- oder Cyanoreste substituierte $-(CH_2)_4-$, $-(CH_2)_5-$,

$-(CH_2)_6-$, $-(CH_2)_7-$, $-CH_2-CH=CH-(CH_2)_2-$, $-(CH_2)_2-O-(CH_2)_2-$, $-(CH_2)_2-S-(CH_2)_2-$, $-NH-(CH_2)_4-$ oder $-NH-CH_2-CH=CH-CH_2$-Gruppe steht und

$R^3$ geradkettiges oder verzweigtes $C_1$-$C_6$-Alkyl, -Alkenyl oder -Alkinyl bedeutet, das durch Halogen, Hydroxy, Cyano, $C_1$-$C_4$-Alkoxy oder -Alkylcarbonyl substituiert sein kann und

Z das Anion einer beliebigen, nicht phytotoxischen Säure HX bedeutet.

2. Verfahren zur Herstellung substituierter Alkylammoniumsalze der Formel I gemäss Anspruch 1, dadurch gekennzeichnet, dass man in an sich bekannter Weise

a) Verbindungen der Formel II

$R^1$ $R^2$

Ar-X-CH-$(CH_2)_n$-CH-Z (II)

in der A, X, $R^1$, n und $R^2$ die oben angegebene Bedeutung haben und Z für eine nucleofuge Abgangsgruppe steht, mit einem tertiären Amin der Formel III

B (III)

in der B die oben angegebenen Bedeutungen hat, umsetzt oder

b) Verbindungen der Formel IV

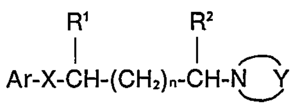

(IV)

in der Ar, X, $R^1$, n, $R^2$ und Y oben genannten Bedeutungen haben, mit Alkylierungsmitteln der Formel V

$R^3$-Z (V)

in der $R^3$ die oben angegebene Bedeutung hat und Z für eine nucleofuge Abgangsgruppe steht, zum Quartärsalz der Formel I alkyliert.

**Revendications**

pour les Etats contractants BE, CH, DE, FR, GB, IT, LU, NL, SE

1. Sels d'alkylammonium substitués de formule I

$R^1$ $R^2$ ⊕

[Ar-X-CH-$(CH_2)_n$-CH-B] [Z] ⊖ (I)

dans laquelle

Ar représente prényle, qui est substitué par un groupe trifluorométhyle, nitro, cyano, $C_2$-$C_4$-alkylcarbonyle, $C_2$-$C_4$-alkylcarbonylamino ou par 2 à 3 des substituants suivants, identiques ou différents: alkyle, alcényle, alkinyle, cycloalkyle, alcoxy, alcényloxy, alkinyloxy, alkylthio, halogénalkyle, halogénalcoxy, halogénalkylthio, alkylcarbonylamino, alkylsulfonyle, alkylaminosulfonyle, dialkylaminosulfonyle ayant chacun jusqu'à 6 atomes C, fluor, chlore, brome, iode, nitro, cyano, aminosulfonyle, phényle ou benzyle

X représente oxygène ou soufre

n est le nombre 0, 1 ou 2

$R^1$ et $R^2$ sont identiques ou différents et représentent hydrogène ou alkyle en $C_1$-$C_4$

B représente un bi-cycle quinuclidine ou pyrrolizidine, ou le groupe 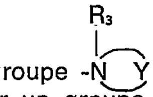, dans lequel

Y est mis pour un groupe $-(CH_2)_4-$, $-(CH_2)_5-$, $-(CH_2)_6-$, $-(CH_2)_7-$, $-CH_2-CH=CH-(CH_2)_2-$, $-(CH_2)_2-O-(CH_2)_2-$, $-(CH_2)_2-S-(CH_2)_2-$, $-NH-(CH_2)_4-$ $-NH-CH_2-CH=CH-CH_2-$ éventuellement substitué par 1 à 3 restes, identiques ou différents, $C_1$-$C_4$-alkyle, -alcoxy, -halogénalkyle, chlore, brome, hydroxy ou cyano,

$R^3$ représente alkyle, alcényle ou alcinyle en $C_1$-$C_6$ à chaîne droite ou ramifiée, qui peut être substitué par halogène, hydroxy, cyano, alcoxy ou alkylcarbonyle en $C_1$-$C_4$,

Z représente l'anion d'un acide HX quelconque, non phytotoxique.

2. Procédé de préparation de sels d'alkylammonium substitués de formule I selon la revendication 1, caractérisé par le fait que

a) on fait réagir des composés de formule II

$R^1$ $R^2$

Ar-X-CH-$(CH_2)_n$-CH-Z (II)

dans laquelle Ar, X, $R^1$, n et $R^2$ ont les significations sus-indiquées, et Z est mis par un groupe de départ nucléofuge, avec une amine tertiaire de formule III

B (III)

où B a les significations sus-indiquées, ou

b) on alkyle, à l'état de sel quaternaire de formule I, des composés de formule IV

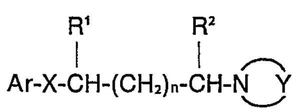

(IV)

dans laquelle Ar, X, $R^1$, n, $R^2$ et Y ont les significations sus-indiquées, avec des moyens d'alkylation de formule V

$R^3$-Z (V)

dans laquelle $R^3$ a la signification sus-indiquée et Z est mis par un groupe de départ nucléofuge.

3. Moyen de régularisation de la croissance des plantes, contenant un ou plusieurs composés selon la revendication 1.

4. Moyen de régularisation de la croissance

des plantes contenant un ou plusieurs composés de la revendication 1 et un support solide ou liquide, ainsi qu'éventuellement un ou plusieurs agents tensio-actifs.

5. Utilisation de composés selon la revendication 1 pour régulariser la croissance des plantes.

6. Procédé de préparation de moyens régularisant la croissance des plantes, caractérisé par le fait qu'on mélange un ou plusieurs composés selon la revendication 1 avec des supports solides ou liquides ainsi qu'éventuellement un ou plusieurs agents tensio-actifs.

**Revendications**
pour l'Etat contractant AT

1. Moyen de régularisation de la croissance des plantes, contenant un sel d'alkylammonium substitué de formule I

$$\left[\begin{array}{l} \quad R^1 \qquad\qquad R^2 \\ \quad | \qquad\qquad\ \ | \\ Ar\text{-}X\text{-}CH\text{-}(CH_2)_n\text{-}CH\text{-}B \end{array}\right]^{\oplus} [Z]^{\ominus} \qquad (I)$$

dans laquelle

Ar représente phényle, qui est substitué par un groupe trifluorométhyle, nitro, cyano, $C_2$-$C_4$-alkylcarbonyle, $C_2$-$C_4$-alkylcarbonylamino ou par 2 à 3 des substituants suivants, identiques ou différents: alkyle, alcényle, alkinyle, cycloalkyle, alcoxy, alcényloxy, alkinyloxy, alkylthio, halogénalkyle, halogénalcoxy, halogénalkylthio, alkylcarbonylamino, alkylsulfonyle, alkylaminosulfonyle, dialkylaminosulfonyle ayant chacun jusqu'à 6 atomes C, fluor, chlore, brome, iode, nitro, cyano, aminosulfonyle, phényle ou benzyle

X représente oxygène ou soufre

n est le nombre 0, 1 ou 2

$R^1$ et $R^2$ sont identiques ou différents et représentent hydrogène ou alkyle en $C_1$-$C_4$

B représente un bi-cycle quinuclidine ou pyrrolizidine, ou le groupe $\overset{R^3}{\underset{|}{}}\!\!-N\frown Y$, dans lequel

Y est mis pour un groupe $-(CH_2)_4-$, $-(CH_2)_5-$, $-(CH_2)_6-$, $-(CH_2)_7-$, $-CH_2-CH=CH-(CH_2)_2-$, $-(CH_2)_2-O-(CH_2)_2-$, $-(CH_2)_2-S-(CH_2)_2-$, $-NH-(CH_2)_4-$, $-NH-CH_2-CH=CH-CH_2-$ éventuellement substitué par 1 à 3 restes, identiques ou différents, $C_1$-$C_4$-alkyle, -alcoxy, -halogénalkyle, chlore, brome, hydroxy ou cyano,

$R^3$ représente alkyle, alcényle ou alcinyle en $C_1$-$C_6$, à chaîne droite ou ramifiée, qui peut être substitué par halogène, hydroxy, cyano, alcoxy ou alkylcarbonyle en $C_1$-$C_4$,

Z représente l'anion d'un acide HX quelconque, non phytotoxique.

2. Procédé de préparation de sels d'alkylammonium substitués de formule I selon la revendication 1, caractérisé par le fait que

a) on fait réagir des composés de formule II

$$\begin{array}{l} \quad R^1 \qquad\qquad R^2 \\ \quad | \qquad\qquad\ \ | \\ Ar\text{-}X\text{-}CH\text{-}(CH_{2n}\text{-}CH\text{-}Z \end{array} \qquad (II)$$

dans laquelle Ar, X, $R^1$, n et $R^2$ ont les significations sus-indiquées, et Z est mis par un groupe de départ nucléofuge, avec une amine tertiaire de formule III

$$B \qquad (III)$$

où B a les significations sus-indiquées, ou

b) on alkyle, à l'état de sel quaternaire de formule I, des composés de formule IV

$$\begin{array}{l} \quad R^1 \qquad\qquad R^2 \\ \quad | \qquad\qquad\ \ | \\ Ar\text{-}X\text{-}CH\text{-}(CH_2)_n\text{-}CH\text{-}N\frown Y \end{array} \qquad (IV)$$

dans laquelle Ar, X, $R^1$, n, $R^2$ et Y ont les significations sus-indiquées, avec des moyens d'alkylation de formule V

$$R^3\text{-}Z \qquad (V)$$

dans laquelle $R^3$ a la signification sus-indiquée et Z est mis par un groupe de départ nucléofuge.

**Claims**
for the contracting states BE, CH, DE, FR, GB, IT, LU, NL, SE

1. A substituted alkylammonium salt of the formula I

$$\left[\begin{array}{l} \quad R^1 \qquad\qquad R^2 \\ \quad | \qquad\qquad\ \ | \\ Ar\text{-}X\text{-}CH\text{-}(CH_2)_n\text{-}CH\text{-}B \end{array}\right]^{\oplus} [Z]^{\ominus} \qquad (I)$$

where

Ar denotes phenyl substituted by trifluoromethyl, nitro, cyano, $C_2$-$C_4$-alkylcarbonyl or $C_2$-$C_4$-alkylcarbonylamino, or by 2 to 3 identical or different substituents selected from the group consisting of alkyl, alkenyl, alkynyl, cycloalkyl, alkoxy, alkenoyloxy, alkylnoloxy, alkylthio, haloalkyl, haloalkoxy, haloalkylthio, alkylcarbonylamino, alkylsulfonyl, alkylaminosulfonyl, dialkylaminosulfonyl, each of a maximum of 6 carbon atoms, fluoro, chloro, bromo, iodo, nitro, cyano, aminosulfonyl, phenyl and benzyl,

X denotes oxygen or sulfur,

n denotes 0, 1 or 2,

$R^1$ and $R^2$ are identical or different and each denotes hydrogen or $C_1$-$C_4$-alkyl,

B denotes bicyclic qunuclidine, bicyclic pyrrolidizine or $\overset{R^3}{\underset{|}{}}\!\!-N\frown Y$,

Y denoting a $-(CH_2)_4-$, $-(CH_2)_5-$, $-(CH_2)_6-$, $-(CH_2)_7-$, $-CH_2-CH=CH-(CH_2)_2-$, $-(CH_2)_2-O-(CH_2)_2-$, $-(CH_2)_2-S-(CH_2)_2-$, $-NH-(CH_2)_4-$ or $-NH-CH_2-CH=$

$=CH-CH_2$-group which is unsubstituted or substituted by from 1 to 3 identical or different radicals selected from the group consisting of $C_1$-$C_4$-alkyl, $C_1$-$C_4$-alkoxy, $C_1$-$C_4$-haloalkyl, chloro, bromo, hydroxy and cyano, and

$R^3$ denoting linear or branched $C_1$-$C_6$-alkyl, $C_1$-$C_6$-alkenyl or $C_1$-$C_6$-alkynyl, each of which is unsubstituted or substituted by halogen, hydroxy, cyano, $C_1$-$C_4$-alkoxy or $C_1$-$C_4$-alkylcarbonyl, and

Z denotes the anion of any nonphytotoxic acid HX.

2. A process for manufacturing substituted alkylammonium salts of the formula I as claimed in claim 1, wherein

a) a compound of the formula II

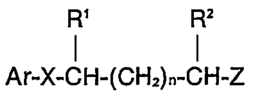 (II)

where Ar, X, $R^1$, n and $R^2$ have the above meanings and Z denotes a nucleofugic leaving group, is reacted in a conventional manner with a tertiary amine of the formula III

B (III)

where B has the above meanings, or

b) a compound of the formula IV

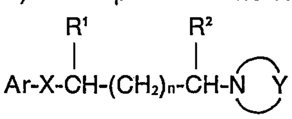 (IV)

where Ar, X, $R^1$, n, $R^2$ and Y have the above meanings, is alkylated in a conventional manner with an alkylating agent of the formula V

$R^3$-Z (V)

where $R^3$ has the above meanings and Z denotes a nucleofugic leaving group, to give a quaternary salt of the formula I.

3. An agent for regulating plant growth, containing one or more compounds as claimed in claim 1.

4. An agent for regulating plant growth, containing one or more compounds as claimed in claim 1 and a solid or liquid carrier, together with, if desired, one or more surfactants.

5. Use of a compound as claimed in claim 1 for regulating plant growth.

6. A process for the manufacture of agents for regulating plant growth, wherein one or more compounds as claimed in claim 1 are mixed with a solid or liquid carrier, together with, if desired, one or more surfactants.

**Claims**

for the Contracting state AT

1. An agent for regulating plant growth, containing a substituted alkylammonium salt of the formula I

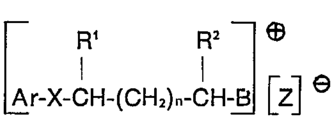 (I)

where

Ar denotes phenyl substituted by trifluoromethyl, nitro, cyano, $C_2$-$C_4$-alkylcarbonyl or $C_2$-$C_4$-alkylcarbonylamino, or by 2 to 3 identical or different substituents selected from the group consisting of alkyl, alkenyl, alkynyl, cycloalkyl, alkoxy, alkenoyloxy, alkynoyloxy, alkylthio, haloalkyl, haloalkoxy, haloalkylthio, alkylcarbonylamino, alkylsulfonyl, alkylaminosulfonyl, dialkylaminosulfonyl, each of a maximum of 6 carbon atoms, fluoro, chloro, bromo, iodo, nitro, cyano, aminosulfonyl, phenyl and benzyl,

X denotes oxygen or sulfur,

n denotes 0, 1 or 2,

$R^1$ and $R^2$ are identical or different anch each denotes hydrogen or $C_1$-$C_4$-alkyl,

B denotes bicyclic quinuclidine, bicyclic pyrrolidine or 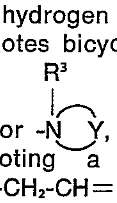,

Y denoting a $-(CH_2)_4-$, $-(CH_2)_5-$, $-(CH_2)_6-$, $-CH_2)_7-$, $-CH_2-CH=CH-(CH_2)_2-$, $-(CH_2)_2-O-(CH_2)_2-$, $-(CH_2)_2-S-(CH_2)_2-$, $-NH-(CH_2)_4-$ or $-NH-CH_2-CH=$ $=CH-CH_2$-group which is unsubstituted or substituted by from 1 to 3 identical or different radicals selected from the group consisting of $C_1$-$C_4$-alkyl, $C_1$-$C_4$-alkoxy, $C_1$-$C_4$-haloalkyl, chloro, bromo, hydroxy and cyano, and

$R^3$ denoting linear or branched $C_1$-$C_6$-alkyl, $C_1$-$C_6$-alkenyl or $C_1$-$C_6$-alkynyl, each of which is unsubstituted or substituted by halogen, hydroxy, cyano, $C_1$-$C_4$-alkoxy or $C_1$-$C_4$-alkylcarbonyl, and

Z denotes the anion of any nonphytotoxic acid HX.

2. A process for manufacturing substituted alkylammonium salts of the formula I as defined in claim 1, wherein

a) a compound of the formula II

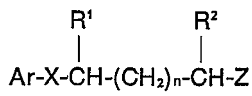 (II)

where Ar, X, $R^1$, n and $R^2$ have the above meanings and Z denotes a nucleofugic leaving group, is reacted in a conventional manner with a tertiary amine of the formula III

B (III)

where B has the above meanings, or

b) a compound of the formula IV

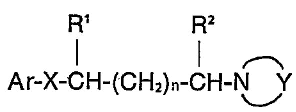 (IV)

where Ar, X, $R^1$, n, $R^2$ and Y have the above meanings, is alkylated in a conventional manner with an alkylating agent of the formula V

$R^3$-Z (V)

where $R^3$ has the above meanings and Z denotes a nucleofugic leaving group, to give a quaternary salt of the formula I.